# EUROPEAN PATENT SPECIFICATION

(11) **EP 1 611 238 B1**
(45) Date of publication and mention of the grant of the patent: **17.06.2009**
(21) Application number: 04714617.0
(22) Date of filing: 25.02.2004
(51) Int. Cl.: C07K 14/47, C12Q 1/68

(54) **GLUTAMATE TRANSPORT MODULATORY COMPOUNDS AND METHODS**
MODULATORISCHE VERBINDUNGEN UND VERFAHREN FÜR GLUTAMATTRANSPORT
COMPOSES MODULANT LE TRANSPORT DU GLUTAMATE ET PROCEDES ASSOCIES

(30) Priority: 26.02.2003 US 450227 P
(43) Date of publication of application: 04.01.2006
(73) Proprietor: The John Hopkins University, Baltimore, MD 21218 (US)
(72) Inventor: ROTHSTEIN, Jeffrey, D., Catonsville, MD 21228 (US)
(74) Representative: Ouzman, Beverley Nicola Claire
(86) International application number: PCT/US2004/005698
(87) International publication number: WO 2004/076675

(56) References cited:
- WO-A2-99/47692
- US-B1- 6 537 803
- NIEDERBERGER E ET AL: "Modulation of spinal nociceptive processing through the glutamate transporter GLT-1" NEUROSCIENCE, NEW YORK, NY, US, vol. 116, no. 1, 15 January 2003 (2003-01-15), pages 81-87, XP004631765 ISSN: 0306-4522
- RD O'SHEA: "Roles and Regulation of Glutamate Transporters in the Central Nervous System" CLINICAL AND EXPERIMENTAL PHARMACOLOGY AND PHYSIOLOGY, vol. 29, 2002, pages 1018-1023, XP002419755
- ROTHSTEIN ET AL.: 'Chronic inhibition of glutamate uptake produces a model of slow neurotoxicity' PROCEEDINGS OF THE NATIONAL ACADEMY OF SCIENCES, USA vol. 90, July 1993, pages 6591 - 6595, XP000907207
- ROWLAND ET AL.: 'Medical prograss: Amyotrophic lateral sclerosis' THE NEW ENGLAND JOURNAL OF MEDICINE vol. 344, no. 22, 31 May 2001, pages 1688 - 1700, XP008041193

### SEQUENCE LISTING

<210> 1 SEQ ID NO : 1
   <211> 4696
   <212> DNA
   <213> Homo sapiens
<400> 1
<210> 2 SEQ ID NO : 2
   <211> 2718
   <212> DNA
   <213> Homo sapiens
<400> 2
   ggtaccttgc tcttggcagt gagcgtgaat gaatggcaca gctcagcccc tgaagcctgt 60
   gtgcagagat tgagggattg tgatggagta gttcattcat gctcatgtta aggggggtgc 120
   taatagcaga ctagtgctcc tgcgattatt aatatctagg tctgggacag attgtgatgg 180
   cttcttttcc agttgccacc tcagcagaaa gggaaataga aaaccctaac ttgtaaagtt 240
   agacaattag actgtaaagt ttgtatatgt gacaacttca gatacaaaga cacacactta 300
   cccttgacgg ggcttaagag gagagtgtca aacataatac caaagtgaaa gaagatagct 360
   cttcatctac aaattatttt taaacacatt taccaggtta aacaataact aatttttcgg 420
   aagagaagag tacccaaagt caaatgccct aagacgaaga gatgcttatg gcattttttt 480
   ttaaataaag aaaatgcaaa gttagagtgg ttctgaagga acctaggatg aataaggtac 540
   agacatgatt attctaatgg tgcagacagg attgagagag aaggggggag gggagagatg 600
   gagaaaggca tggatggaag atgacgtttg gattcagatt ttggaaagga gagtaaagga 660
   aggaggtaag cagagattta ttttttaaat tttattaatg tgttttcccc tctttttctt 720
   gttatttttc tcatctgtct gttcatactt ggatattttg tccaataaac tatcttctaa 780
   ggactctgaa aatgcactga atatttttgg agggtttact ggggtgccag acgccacttt 840
   aggagtttta catatcctct ccatttcatt tagttctctt agcacagaga agtgggagaa 900
   gatagtccca ttttacaggt gggatgaaga gagagatgga ggaatttgcc ccaggttact 960
   cagctagaag gtggtgaaga actcaagcct tcggatatca gcgcctggca tttaactacc 1020
   aatcggtcct gctgggactc cggctcctct ggcaccatcc ccgggaccta ctcagagagt 1080
   ttgcacgtgg ccggtcgcgt tccatcgtct aacaaggtcc agcacagcgc aaatccgaag 1140
   atcgtctacc ccggggaaaa agagagtctg tttaattctc ctgtggccct ccaagtgagt 1200
   tcttttgggt tccattgcct agacgaggaa agtgaggctt tgcctgctct gcgctcacag 1260
   ggtcggcaag tagtgggacc ctaggttcct gcagtattcc agagataatc aaagctgcac 1320
   aggtctcgtc atttttatgc aaaggcgtcc ggaaggctcg aactctccct tgcacaagcc 1380
   catctgtctc tgtgcgccgc ccccgggaca cggaagcagg cggcgagcag cgccgagtgg 1440
   gtggagaacc gtcccccgcc actcacccct cggccaactc tccgcgcctt ctcagccggc 1500
   acccacgagg ccgacctctc tcggcctaaa aaaaaaaaaa aaaaatcccg gcctcccctg 1560
   caccccgccc gccgccccca gggagctgca ttaatattaa tctcgctgaa taattgaagg 1620
   ccagagattt attcgagctt cggcggggga gggagcgcag ctgggccgcg tttaggctgc 1680
   accacccgcg tgtttcagcc gctcgactcc gctggacctg ggacccccag acgtgggagg 1740
   atggggtggg tgtgcctgcc tgtgagtttg gggtgagtgg tgagctgaag cgggtgctcc 1800
   ggggagtgag gagggagcgc caggggctgc tccagggagg cggagacgga ggggcatccc 1860
   gggtctccgc gcggtcgcct gcgcttcacc ccgcacgggg tgacctgggg ccacgcgggc 1920
   ttcaggggaa acaatagcta ctccttagat cctgggctcc tgccaccggc tgcccaagcc 1980
   ttcccggacg agcggcgggg cctcttttct tatttggcta atttatggcg agaggctggg 2040
   ggaagggatg gcagaggagg gaccgcgact gaaaatgggg gcggggggcg gcggttaaag 2100
   gagttgcccg aggcggcggc gcgggtgatg tcagctctcg acgaaaatag agagggatcg 2160
   cctgcaaatc cccagctccg gcggggctaa accttgcaat ccctccctgg ccggcgccga 2220
   gccagagcgc agcggcctcc accgcctccc caggcgcgca cacacccgca cacgcgcacg 2280
   cacgctcacc gtcctctgcc accactctct gctcccgcca ctcgccgcgc ccgcgagccc 2340
   cgcagcaaag cacaggtggc agcggctgca ggggcgcatc gccggcgtgc gccctcctgc 2400
   agccctgggc gcatcgctct ctcggggaag ccaccctcgg agcccccgga gctccccgcc 2460
   aagcgccatc cccgcgggcg gaggggagcg cgggtcgcgc gccgtggaga gccgggacgc 2520
   ggattagcgc ccgcaggagc ctcctgcgcc cgttgaggcg ctaaagggct taccccggag 2580
   gcgggtggaa gggcgggcag aggctcctct taaataccgc tcccggccgc acttcgcgct 2640
   caccccggcg tccgctttct ccctcgccca cagctgccgg atagtgctga agaggagggg 2700
   gcgttcccca gaccatgg 2718
<210> 3 SEQ ID NO : 3
   <211> 2454
   <212> DNA
   <213> Homo sapiens
<400> 3
   ggtaccttgc tcttggcagt gagcgtgaat gaatggcaca gctcagcccc tgaagcctgt 60
   gtgcagagat tgagggattg tgatggagta gttcattcat gctcatgtta aggggggtgc 120
   taatagcaga ctagtgctcc tgcgattatt aatatctagg tctgggacag attgtgatgg 180
   cttcttttcc agttgccacc tcagcagaaa gggaaataga aaaccctaac ttgtaaagtt 240
   agacaattag actgtaaagt ttgtatatgt gacaacttca gatacaaaga cacacactta 300
   cccttgacgg ggcttaagag gagagtgtca aacataatac caaagtgaaa gaagatagct 360
   cttcatctac aaattatttt taaacacatt taccaggtta aacaataact aatttttcgg 420
   aagagaagag tacccaaagt caaatgccct aagacgaaga gatgcttatg gcattttttt 480
   ttaaataaag aaaatgcaaa gttagagtgg ttctgaagga acctaggatg aataaggtac 540
   agacatgatt attctaatgg tgcagacagg attgagagag aaggggggag gggagagatg 600
   gagaaaggca tggatggaag atgacgtttg gattcagatt ttggaaagga gagtaaagga 660
   aggaggtaag cagagattta ttttttaaat tttattaatg tgttttcccc tctttttctt 720
   gttatttttc tcatctgtct gttcatactt ggatattttg tccaataaac tatcttctaa 780
   ggactctgaa aatgcactga atatttttgg agggtttact ggggtgccag acgccacttt 840
   aggagtttta catatcctct ccatttcatt tagttctctt agcacagaga agtgggagaa 900
   gatagtccca ttttacaggt gggatgaaga gagagatgga ggaatttgcc ccaggttact 960
   cagctagaag gtggtgaaga actcaagcct tcggatatca gcgcctggca tttaactacc 1020
   aatcggtcct gctgggactc cggctcctct ggcaccatcc ccgggaccta ctcagagagt 1080
   ttgcacgtgg ccggtcgcgt tccatcgtct aacaaggtcc agcacagcgc aaatccgaag 1140
   atcgtctacc ccggggaaaa agagagtctg tttaattctc ctgtggccct ccaagtgagt 1200
   tcttttgggt tccattgcct agacgaggaa agtgaggctt tgcctgctct gcgctcacag 1260
   ggtcggcaag tagtgggacc ctaggttcct gcagtattcc agagataatc aaagctgcac 1320
   aggtctcgtc atttttatgc aaaggcgtcc ggaaggctcg aactctccct tgcacaagcc 1380
   catctgtctc tgtgcgccgc ccccgggaca cggaagcagg cggcgagcag cgccgagtgg 1440
   gtggagaacc gtcccccgcc actcacccct cggccaactc tccgcgcctt ctcagccggc 1500
   acccacgagg ccgacctctc tcggcctaaa aaaaaaaaaa aaaaatcccg gcctcccctg 1560
   caccccgccc gccgccccca gggagctgca ttaatattaa tctcgctgaa taattgaagg 1620
   ccagagattt attcgagctt cggcggggga gggagcgcag ctgggccgcg tttaggctgc 1680
   accacccgcg tgtttcagcc gctcgactcc gctggacctg ggacccccag acgtgggagg 1740
   atggggtggg tgtgcctgcc tgtgagtttg ggggtgagtg tgagctgaag cgggtgctcc 1800
   ggggagtgag gagggagcgc caggggctgc tccagggagg cggagacgga ggggcatccc 1860
   gggtctccgc gcggtcgcct gcgcttcacc ccgcacgggg tgacctgggg ccacgcgggc 1920
   ttcaggggaa acaatagcta ctccttagat cctgggctcc tgccaccggc tgcccaagcc 1980
   ttcccggacg agcggcgggg cctcttttct tatttggcta atttatggcg agaggctggg 2040
   ggaagggatg gcagaggagg gaccgcgact gaaaatgggg gcggggggcg gcggttaaag 2100
   gagttgcccg aggcggcggc gcgggtgatg tcagctctcg acgaaaatag agagggatcg 2160
   cctgcaaatc cccagctccg gcggggctaa accttgcaat ccctccctgg ccggcgccga 2220
   gccagagcgc agcggcctcc accgcctccc caggcgcgca cacacccgca cacgcgcacg 2280
   cacgctcacc gtcctctgcc accactctct gctcccgcca ctcgccgcgc ccgcgagccc 2340
   cgcagcaaag cacaggtggc agcggctgca ggggcgcatc gccggcgtgc gccctcctgc 2400
   agccctgggc gcatcgctct ctcggggaag ccaccctcgg agcccccgga gctc       2454
<210> 4 SEQ ID NO : 4
   <211> 861
   <212> DNA
   <213> Homo sapiens
<400> 4
   cccgggtctc cgcgcggtcg cctgcgcttc accccgcacg gggtgacctg gggccacgcg 60
   ggcttcaggg gaaacaatag ctactcctta gatcctgggc tcctgccacc ggctgcccaa 120
   gccttcccgg acgagcggcg gggcctcttt tcttatttgg ctaatttatg gcgagaggct 180
   gggggaaggg atggcagagg agggaccgcg actgaaaatg ggggcggggg gcggcggtta 240
   aaggagttgc ccgaggcggc ggcgcgggtg atgtcagctc tcgacgaaaa tagagaggga 300
   tcgcctgcaa atccccagct ccggcggggc taaaccttgc aatccctccc tggccggcgc 360
   cgagccagag cgcagcggcc tccaccgcct ccccaggcgc gcacacaccc gcacacgcgc 420
   acgcacgctc accgtcctct gccaccactc tctgctcccg ccactcgccg cgcccgcgag 480
   ccccgcagca aagcacaggt ggcagcggct gcaggggcgc atcgccggcg tgcgccctcc 540
   tgcagccctg ggcgcatcgc tctctcgggg aagccaccct cggagccccc ggagctcccc 600
   gccaagcgcc atccccgcgg gcggagggga gcgcgggtcg cgcgccgtgg agagccggga 660
   cgcggattag cgcccgcagg agcctcctgc gcccgttgag gcgctaaagg gcttaccccg 720
   gaggcgggtg gaagggcggg cagaggctcc tcttaaatac cgctcccggc cgcacttcgc 780
   gctcaccccg gcgtccgctt tctccctcgc ccacagctgc cggatagtgc tgaagaggag 840
   ggggcgttcc ccagaccatg g                                           861

## Description

### Related Applications

This application claims the benefit of U.S. Provisional Application Serial No. 60/450,227, filed February 26, 2003. The entire contents of this application is incorporated herein by this reference.

### Government Support

This work described herein was supported by a grant from the National Institutes of Health (Grant No. NS33958). Therefore, the U.S. Government may have certain, rights in the invention.

### Background of the Invention

Neurological disorders can significantly impact the central nervous system (CNS) and motor neuron units. For example, certain neurological disorders of the CNS are known to adversely affect the brain and associated structures. Neurological disorders affecting motor neuron units have been grouped into motor neuron diseases and peripheral neuropathies. See generally Kandel, E.R. et al; (1991) in Principles of Neuroscience, Appleton & Lange, Norwalk, CT; and Rowland, L.P. (ed.) (1982) in Human Motor Neuron Diseases. New York. Raven Press.

An illustrative motor neuron disease is amyotrophic lateral sclerosis (ALS). ALS has been reported to be a chronic neuromuscular disorder having recognized clinical manifestations. For example, it has been suggested that degeneration of cortical and spinal/bulbar motor neurons may play a key role in the disorder. ALS is nearly always fatal. About 95% of all ALS cases are sporadic, with many of the remaining cases showing autosomal dominant inheritance. See e.g., Kuncl R.W. et al., (1992) Motor Neuron Diseases In Diseases of the Nervous System, Asbury et al. eds. (Philadelphia W.B.Saunders) pp. 1179-1208; Brown, R.H., (1996) Amer. Neurol. 30:145; Siddique, T. and Deng., H.X. (1996) Hum. Mol. Genetics 5:1465).

Specific CNS disorders have been also described. In particular, some have been attributed to cholinergic, dopaminergic, adrenergic, serotonergic deficiencies or combinations thereof. CNS disorders of severe impact include pre-senile dementia (sometimes referred to as Alzheimer's disease (AD) or early-onset Alzheimer's disease), senile dementia (dementia of the Alzheimer's type), Parkinson's disease (PD), and Huntington's disease (HD, sometimes referenced as Huntington's chorea). Such CNS disorders are well-represented in the human population. See generally; Gusella, J.F. et al. (1983) Nature 306: 234; Borlauer. W. and Jprmuloewoca. P. (eds.) (1976); Adv. in Parkinsonism: Biochemistry, Physiology, Treatment. Fifth International Symposium on Parkinson's Disease (Vienna) Basel: Roche; and references cited therein.

Significant attention has been directed towards understanding the etiology of motor neuron diseases. For example, abnormal levels of certain excitotoxic neurotransmitters have been reported to adversely contribute to many motor neuron diseases. In particular, glutamate-mediated excitotoxicity is recognized to have a critical role in ALS. See e.g., Rothstein J.D. et al., (1990) Ann. Neurol. 28: 18. ; Rothstein J.D. et al.(1992) N. Engl. Med. 326: 1464; Rothstein J.D., et al. (1993) PNAS (USA) 90: 6591; and Lacomblez , L. et al., (1996) Lancet 347: 1179.

The astroglial transporters GLAST (EAAT1) and GLT-1(EAAT2) are responsible for the largest percentage of glutamate transport in the forebrain. As such, they both represent intriguing targets for modulation of expression, and thereby as agents to retard-disease progression, including neurodegeneration, seizure, and brain tumor growth.

There has been substantial efforts towards understanding mechanisms for reducing glutamate levels in the nervous system. For example, high-affinity, sodium-dependent glutamate transport is one reported means of inactivating glutamate. In particular, astrocytic excitatory amino acid transporter 2 (EAAT2) proteins are believed to have substantial functions in that inactivation. See e.g., Rothstein J.D. et al.. (1994) Neuron 28: 18; Rothstein J.D. et al., (1995) Ann. Neurol. 38: 78. and references cited therein.

In particular, investigations have suggested that EAAT2 is a predominant glutamate transporter. More particularly, certain antisense knockdown studies have been reported to demonstrate that EAAT2-loss can lead to excitotoxic neuronal degeneration and progressive motor impairment. Studies of ALS and other neurodegenerative disorders have related impaired glutamate transport to loss of the EAAT2 protein. In particular, up to 60% to 70% of the sporadic ALS patients examined have a 30% to 95% loss of the EAAT2 protein. See e.g., Haugeto et al., *supra;* Rothstein J.D., et al., (1996) Neuron 16:675; Bristol, L.A. and Rothstein, J.D. (1996) Ann. Neurol. 39:676.

There have been attempts to treat or prevent neurological disorders of the CNS and the motor neuron units. However, most existing therapies do not always stem the development or severity of the disorders in afflicted patients. See e.g., Rowell, (1987) Adv. Behav. Biol. 31:191; Rinne, et al. Brain Res. (1991) 54:167; U.S. Pat. No. 5,210,076 to Berliner; Yurek, D.M. (1990) Ann. Rev. Neurosci. 13:415, and Rowland et al. *supra.*

Niederberger et al., (Neuroscience 116 (2003):81-87) shows nociceptive stimulation increases expression of the glumate transporter GLT-1.

O'Shea (Clin. Expt. Pharm. and Physiology (2002) 29:1018-1023) reviews the roles of glutamate transporters in the CNS and notes that excitatory amino acid transporters are attractive targets for drugs.

Accordingly, there is a need in the field for effective therapies for treating neurological disorders.

### Summary of the Invention

Described herein is the use of an excitatory amino acid transporter (EAAT) expression promoting agent that is an antibiotic, in the manufacture of a medicament for treating a neurological disease or disorder associated with altered glutamate transmission.

In one aspect, the antibiotic is a compound identified by a screening assay including the steps of
a) contacting a test antibiotic with a nucleic acid molecules comprising a cDNA molecule and having a nucleotide sequence which is at least about 60% identical to the nucleotide sequence of SEQ ID NO: 1,2, 3, or 4, wherein the nucleic acid molecule is capable of directing mRNA expression from a promoterless reporter vector, or a complement thereof, or a cell comprising said nucleic acid molecule; and
b) determining whether mRNA expression or the polypeptide encoded by the cDNA is modulated, thereby identifying an antibiotic which is an EAAT2 expression promoting agents.

The EAAT2 protein expression can be increased *in vivo* or EAAT2 protein expression can be increased *in vitro.*

Other aspects of the invention are those wherein the EAAT2 expression promoting agent comprises at least one structural clement selected from heterocycles having at least one ring sulfur atom, tertiary amines, quaternary ammonium salts, steroids, polyols, polyketide, guanidine, urea, or arsenate; those wherein the EAAT2 expression promoting agent increases EAAT2 production by 200% or more relative to non-regulated production; wherein the EAAT2 expression promoting agent increases EAAT2 production by 300% or more relative to non-regulated production; wherein the EAAT2 expression promoting agent increases EAAT2 production by 600% or more relative to non-regulated production.

In another aspect, the EAAT2 expression promoting agent is selected from the group consisting of penicillin V potassium, dequalinium chloride, quinapril, amoxicillin, pridinol methansulfonate, aklomide, vancomycin hydrochloride, thiamphenicol, ceftriaxone sodium, 1,3-dipropyl-8-cyclopentylxanthine (DPCPX), cephapirin sodium, actinospectacin, cefoperazone sodium, acivicin, acetylcholine, chloramphenicol, vidarabine, atenolol, oxytetracycline, glafenine, oxymetazoline hydrochloride, gallamine, perillic acid (-), amitriptyline hydrochloride, tetracaine hydrochloride, disopyramide phosphate, sisomicin sulfate, ketamine , hydrochloride, xylazine, bicuculline, flurbiprofen, cefadroxil, bacampicillin hydrochloride, tiapride hydrochloride, norethindrone acetate, bergaptene, carisoprodol, citiolone, piroxicam, erythromycin ethylsuccinate, furegrelate sodium, albendazole, dihydrostreptomycin sulfate, aloin, fenoprofen, flutamide, ampicillin sodium, amprolium, sparteine sulfate, medroxyprogesterone acetate, alexidine hydrochloride, clindamycin hydrochloride, cephalothin sodium, daidzein, meclizine hydrochloride, lindane, bromopride, N-(3-trifluoromethylphenyl)piperazine hydrochloride (TFMPP), enoxotone, ipratropium bromide, bufexamac, gluconolactone, rifampin, hydroxychloroquine, coleoforsin, chloroxine, oxidopamine hydrochloride, camptothecin, nafcillin sodium, mianserin hydrochloride, acetarsol, prilocaine hydrochloride, deferoxamine mesylate, hexamethonium bromide, methenamine, paraxanthine, harmalol hydrochloride, pyrithione zinc, hydrocortisone butyrate, acetazolamide, aminoglutethimide, meclofenoxate hydrochloride, 2-phenpropylamino-5-nitrobenzoic acid (NPPB), amiodarone hydrochloride, aconitine, hydroxyprogesterone caproate, and diosmin.

In another aspect, the EAAT2 expression promoting agent is selected from the group consisting of penicillin V potassium, dequalinium chloride, quinapril, amoxicillin, pridinol methansulfonate, aklomide, vancomycin hydrochloride, thiamphenicol, ceftriaxone sodium, 1,3-dipropyl-8-cyclopentylxanthine (DPCPX), cephapirin sodium, actinospectacin, cefoperazone sodium, acivicin, acetylcholine, chloramphenicol, vidarabine, cefoperazone sodium, acivicin, acetylcholine, chloramphenicol, vidarabine, atenolol, oxytetracycline, glafenine, and oxymetazoline hydrochloride; those wherein the EAAT2 expression promoting agent is selected from the group consisting of penicillin V potassium, dequalinium chloride, quinapril, amoxicillin, pridinol methansulfonate, aklomide, and vancomycin hydrochloride; those wherein the EAAT2 expression promoting agent is selected from the group consisting of penicillin V potassium, dequalinium chloride, and quinapril.

The EAAT2 expression promoting agent can be used for decreasing extracellular glutamate concentration in a mammal, wherein at least one EAAT2 expression promoting agent is administered to the mammal. The expression promoting agent can be an antibiotic identified by a screening assay including the steps of:
a) contacting a cell that expresses EAAT2 with a test antibiotic; and
b) determining whether expression of the EAAT2 in the cell is modulated in the presence of the test antibiotic relative to the absence of the test antibiotic, thereby identifying an antibiotic which modulates expression of EAAT2.

The EAAT2 protein expression can be increased *in vivo* or the EAAT22 protein expression can be increased *in vitro.* In these methods, the EAAT2 expression promoting agent is an antibiotic, and the EAAT2 expression promoting agent can optional comprise at least one structural element selected from heterocycles having at least one ring sulfur atom, tertiary amines, quaternary ammonium salts, steroids, polyols, polyketide, guanidine, urea, or arsenate.

In another aspect, the EAAT2 expression promoting agent can be used wherein the mammal is a primate; and wherein the mammal is a human. The extracellular glutamate concentration can be reduced by at least about 50% relative non-regulated concentration; or the extracellular glutamate concentration can be reduced by at least about 75% relative non-regulated concentration.

The disease or disorder associated with altered glutamate transmission can be a neurological disease (e.g., Parkinson's disease, Huntington's disease, Alzheimer's disease, multiple sclerosis, amyotrophic lateral sclerosis, acute neurological diseases, epilepsy, spinal cord injury, brain trauma, glaucoma, or psychiatric disorders. The method can be those wherein the EAAT2 expression promoting agent is a compound identified by a screening assay as described above.

In another aspect, the invention relates to an antibiotic which is a compound identified by a screening assay including the steps of
a) contacting a cell that expresses EAAT2, with a test antibiotic; and
b) determining whether expression of the EAAT2 in the cell is modulated in the presence of the test antibiotic compared to in the absence of the test antibiotic, thereby identifying an antibiotic which modulates expression of the EAAT2 as a compound which is capable of treating a neurological disease or disorder.

The EAAT2 expression promoting agent can be a β-lactam antibiotic; or the EAAT2 expression promoting agent can be a penicillin class, cephalosporin class, carbapenam class or monobactam class compound.

Another aspect is wherein the neurological disease or disorder associated with altered glutamate transmission is associated with learning or memory, or enhancing learning, memory; or cognitive enhancement.

The subject (including a subject identified as in need of such treatment) can be administered with an effective amount of an antibiotic described herein, or a composition described herein to produce such effect. Identifying a subject in need of such treatment can be in the judgment of a subject or a health care professional and can be subjective (e.g. opinion) or objective (e.g. measurable by a test or diagnostic method).

Table 1 lists compounds (or salts or solvates thereof) useful in the methods delineated herein.

**Table 1-β-lactam compounds**

| **Penicillins** | **Cephalosporins and cephamycins** | **Other Beta Lactams** |
|---|---|---|
| benzylpenicillin (penicillin g) | cefaclor | Aztreonam |
| procaine benzylpenicillin | cefadroxil | Imipenem |
| (procaine penicillin) | cefadyl | Meropenem |
| phenoxymethylpenicillin | cefalexin | Ertapenem |
| (penicillin v) | cefamandole | FK-037 |
| benzathine penicillin | cefazolin | |
| hetacillin | cefditoren | |
| cloxacillin | cefepime | |
| carbenicillin | cefetamet | |
| flucloxacillin | cefdinir | |
| ampicillin | cefixime | |
| amoxicillin | cefizox | |
| co-amoxiclav | cefotaxime | |
| carboxypenicillin | cefinetazole | |
| ticarcillin | cefobid | |
| timentin | cefonicid | |
| tazocin (ureidopenicillin | cefoperazone | |
| piperaciliin with the beta- | cefotan | |
| lactamase inhibitor tazobactam) | cefotetan | |
| piperacillin | cefoxitin | |
| pivmecillinam | cefpirome | |
| amoxicillin-clavulanate | cefpodoxime | |
| piperacillin | cefpodoxime proxetil | |
| oxacillin | cefprozil | |
| | cefradine | |
| | ceftazidime | |
| | ceftibuten | |
| | ceftidoren | |
| | ceftin | |
| | ceftizoxime | |
| | ceftriaxone | |
| | cefuroxime | |
| | cefuroxime axetil | |
| | cephalexin | |
| | cefzil | |
| | cephalothin | |

### Brief Description of the Drawings

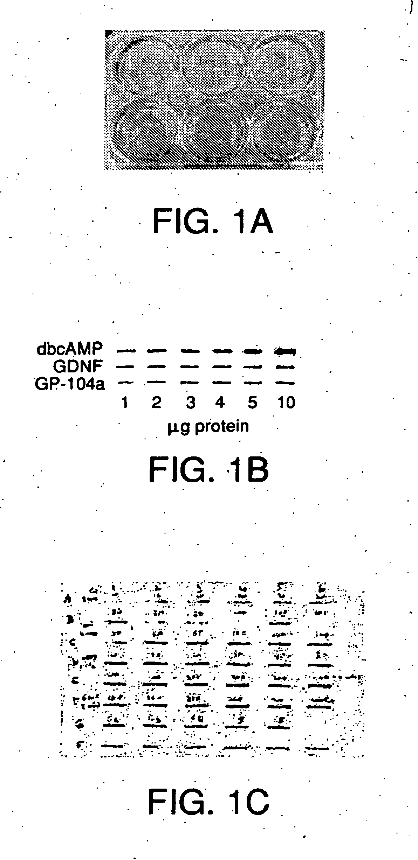

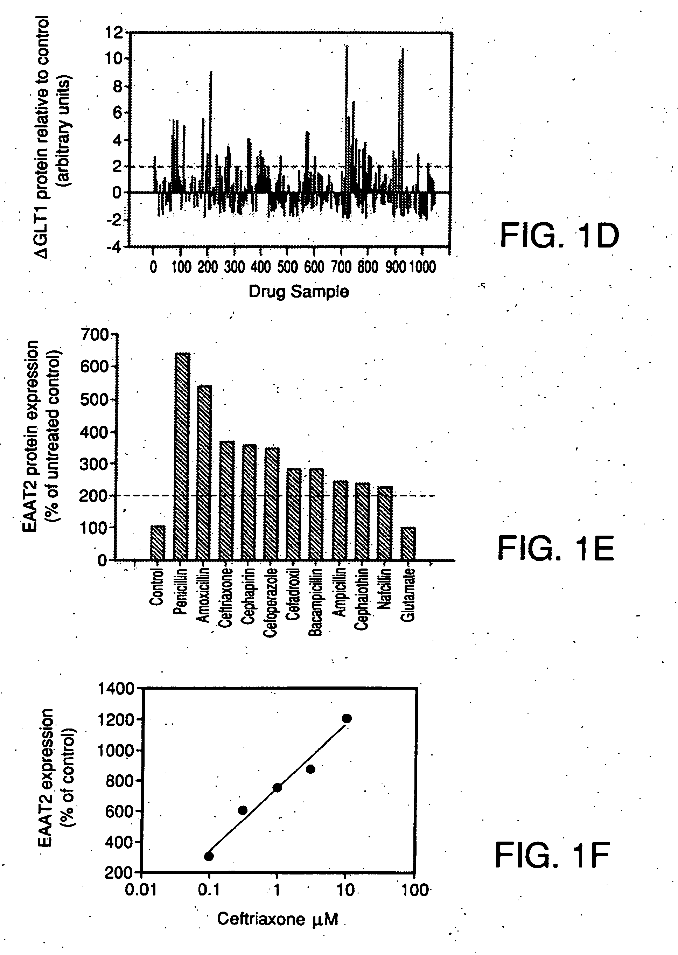

FIG.1, comprising FIG. 1A through FIG. 1F, is a series of images demonstrating that β-lactam antibiotics are inducers of EAAT2 protein based on a screen of 1040 PDA approved drugs. FIG. 1A shows spinal cord cultures incubated with test compound; FIG. 1B is a sample slot blot from tissue homogenates; FIG. 1C illustrates a representative screening slot blot; FIG. 1D illustrates screening results of a library of test compounds; FIG. 1E is an illustration of expression results from treatment with various compounds categorized by classes; FIG. 1F shows a dose-response analysis of EAAT2 expression for ceftriaxone.

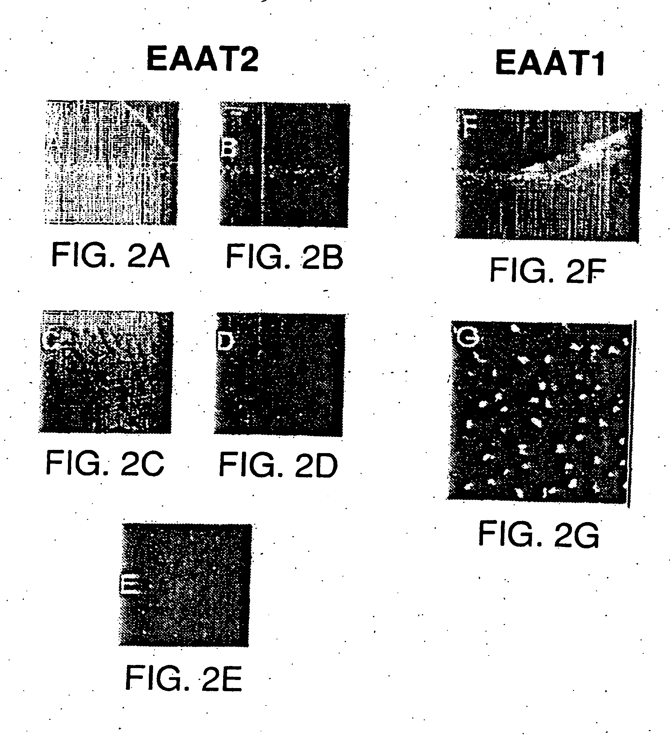

FIG. 2, comprising FIG. 2A through FIG. 2G, is a series of images demonstrating the generation of promoter reporter transgenic mice. FIG. 2A-2E illustrate expression of EAAT2 promoter fragments in mouse brain at two weeks and widespread expression of the reporter in astrocytes throughout the brain parenchyma. FIG. 2F shows astrocytes from EAAT1 promoter reporter, and FIG. 2G shows cortical expression of EAAT1 Bac-eGFP reporter, in transgenic mice.

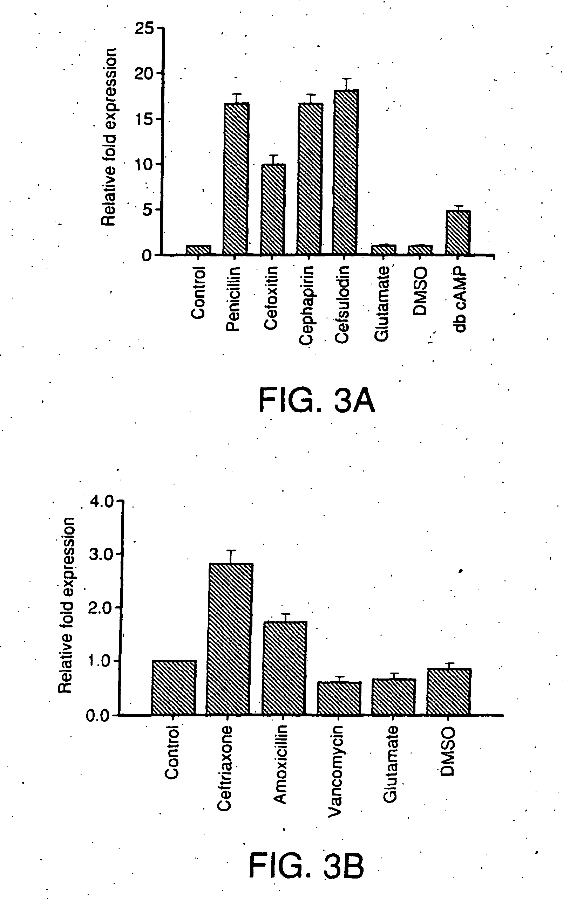

FIG. 3, comprising FIG. 3A and FIG. 3B, is a series of images demonstrating that β-lactams activate EAAT2 promoter. FIG. 3A shows activation (by compound class) of EAAT2 promoter by various test compounds in Cos7 cells and FIG. 3B shows activation (by compound class) of EAAT2 promoter in human astrocytes transfected with the EAAT2 promoter-eGFP reporter. β-lactam antibiotics (10µM) markedly activate the EAAT2 promoter, while controls such as glutamate has no effect. The known activator, dibutyryl cyclic AMP, has a consistent, but smaller effect.

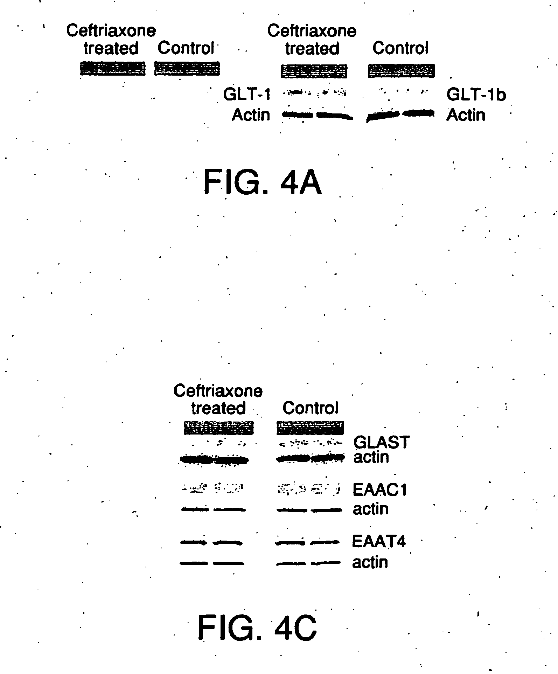

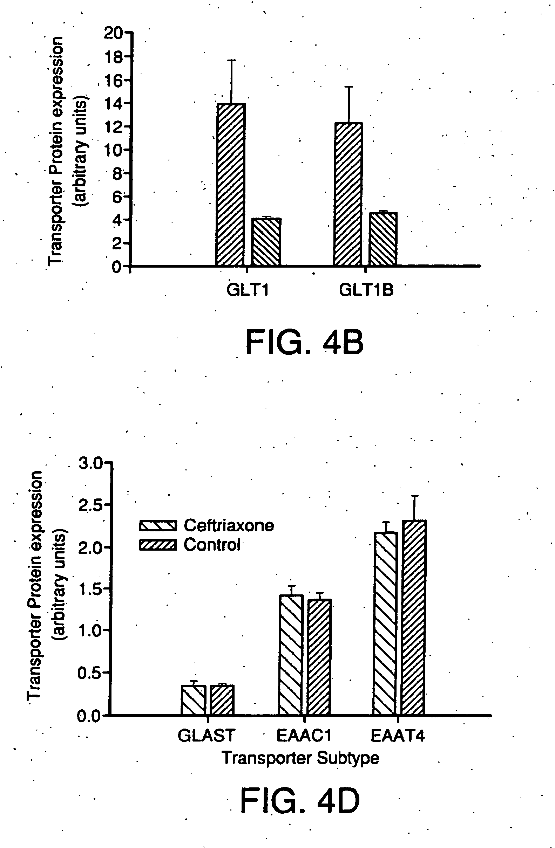

FIG. 4, comprising FIG. 4A through FIG. 4D, demonstrates that ceftriaxone induces expression of GLT1 and GLT1b, but not other proteins, in vivo. Rats were injected (ip) daily for 5 days with ceftriaxone (200mg/kg) a dose known to produce low micromolar brain concentrations. FIG. 4A is a western blot of ceftriaxone effect on GLT-1 and GLT-1B expression in the hippocampal; FIG. 4B illustrates the effect of ceftriaxone on GLT-1 and GLT-1B expression in the hippocampal; FIG. 4C is a western blot of ceftriaxone effect on GLAST, EAAC1 and EAAT4 expression; FIG. 4D illustrates the effect of ceftriaxone on GLAST, EAAC1 and EAAT4 expression.

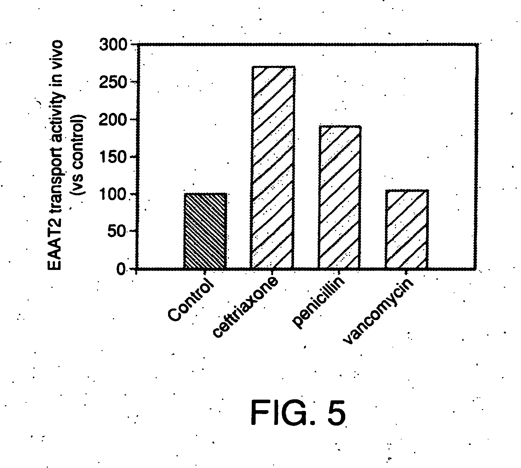

FIGS. 5 illustrates the effect of various antibiotics on glutamate transport. FIG. 5 demonstrates that β-lactam antibiotic ceftriaxone and penicillin administration leads to a functional increase in glutamate transport. Daily treatment with ceftriaxone or penicillin (200mg/kg, 5 days) not only increased GLT1 protein, but also increased GLT1-mediated glutamate transport, as determined by ³H-glutamate transport assays (in the presence/absence of dihydrokainate, to measure GLT1-specific transport). It was observed that the non-β-lactam antibiotic vancomycin, did not increase glutamate transport activity.

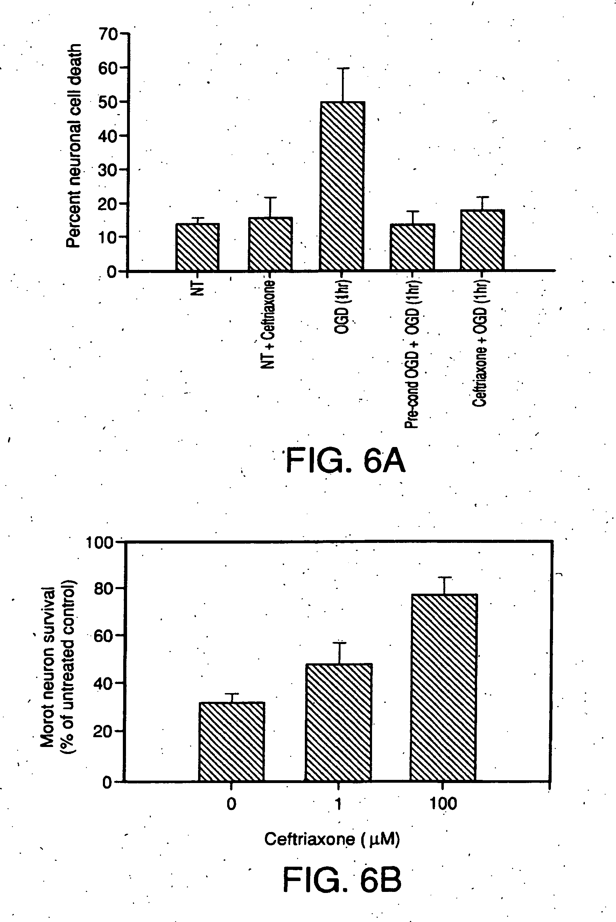

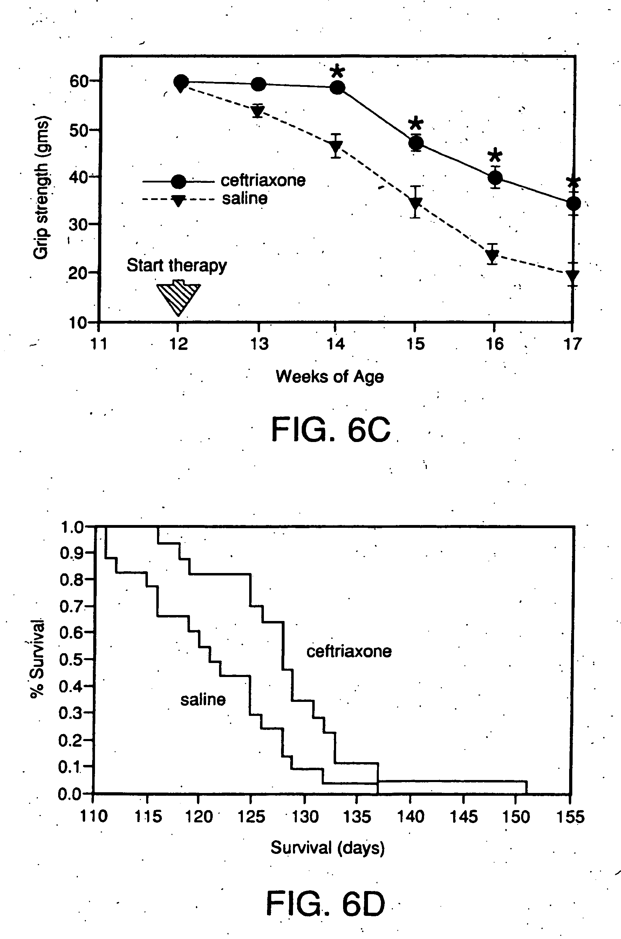

FIG. 6, comprising FIG.6A through FIG. 6D, demonstrates neuroprotection by ceftriaxone. FIG. 6A illustrates the effect of ceftriaxone on ischemic tolerance. It was observed that oxygen glucose derivation (OGD) of cortical neurons lead to reliable cell death; while preconditioning with brief OGD was protective. Similar protection was afforded by ceftriaxone (1µM) pretreatment. FIG. 6B illustrates the effect of ceftriaxone on motor neuron degeneration. It was observed that ceftriaxone prevented motor neuron degeneration in vitro. Chronic treatment of spinal cord organotypic cultures with the glutamate transport inhibitor threo-hydroxyaspartate lead to loss of >50% motor neurons.

Co-treatment with ceftriaxone prevented this excitotoxic loss of motor neurons. FIG. 6C illustrates the effect of ceftriaxone on grip strength (in vivo model); FIG. 6D illustrates the effect of ceftriaxone on survival in G93A SOD1 ALS mice. It was observed that ceftriaxone therapy (200mg/kg daily i.p.) delayed loss of muscle strength in G93A SOD1 mice. Therapy was initiated at disease onset (approx 12 wks age). Asterisks indicate significant difference from saline control (P<0.05) at each time point.

### Detailed Description of the Invention

The present invention is based, at least in part, on the discovery of the sequence of the EAAT2 promoter. Accordingly, the present invention provides use of an antibiotic in the manufacture of a medicament for treating a neurological disease or disorder associated with altered glutamate transmission, and screening assays to identify suitable antibiotics.

The acidic amino acids glutamate (Glu) and aspartate are the predominant excitatory neurotransmitters in the mammalian central nervous system (CNS). Although there are millimolar concentrations of these excitatory amino acids (EAAs) in the brain, extracellular concentrations are maintained in the low micromolar range to facilitate crisp synaptic transmission and to limit the neurotoxic potential of these EAAs. A family of Na⁺ -dependent high affinity transporters is responsible for the regulation and clearance of extracellular EAAs.

Glutamate and aspartate activate ligand-gated ion channels that are named for the agonists N-methyl-D-aspartate (NMDA), a-amino-3-hydroxy-5-methyl-4-isoxazolepropionate (AMPA), and kainate. These ionotropic EAA receptors mediate rapid synaptic depolarization and are important for a number of other physiological processes, including synaptic plasticity and synapse development. The EAAs also activate a family of metabotropic receptors coupled through G-proteins to second messenger systems or ion channels. It is well established that the EAAs are extremely important for normal brain function. However, there is substantial evidence that an extracellular accumulation of EAAs and excessive activation of EAA receptors also contributes to the neuronal cell death observed in acute insults to the CNS. The process known as, 'excitotoxicity', may also contribute to neuronal loss observed in chronic neurodegenerative diseases, including amyotrophic lateral sclerosis (ALS).

The intracellular concentrations of glutamate (5-10 mM) and aspartate (1-5 mM) are 1000-fold to 10,000-fold greater than the extracellular concentrations (<1-10 µM). Unlike many other neurotransmitters, there is no evidence that glutamate or aspartate is metabolized extracellularly. Instead, they are cleared from the extracellular space by transport into neurons and astrocytes.

Several subtypes of Na⁺-dependent glutamate transporters have been identified through pharmacological strategies and cDNA cloning. Five known distinct cDNA clones that express Na⁺-dependent high-affinity glutamate transport are referred to herein as GLT-1/EAAT2, EAAC1/EAAT3, GLAST/EAAT1, EAAT4, and EAAT5. There is also evidence for additional heterogeneity of GLT-1 and GLAST that originates from alternate mRNA splicing.

Expression of two of these transporters, GLT-1 and GLAST, is generally restricted to astroglia. Expression of two other transporters, EAAC1 and EAAT4, is generally restricted to neurons, and EAAT5 is thought to be restricted to retina. Of the three transporters found in forebrain (GLT-1, GLAST, and EAAC1), GLT-1 appears to be the only transporter that is specific to brain tissue, suggesting that GLT-1 expression is controlled by brain specific mechanisms.

Previously, it was thought that presynaptic transporters had a major role in the clearance of EAAs during synaptic transmission. This was based on the evidence that activity is enriched 2-fold in synaptosomal membrane preparations compared to fractions enriched in mitochondria or myelin. However, it is now known that these membrane preparations contain resealed glial membranes and tremendous amounts of GLT-1 protein. In addition, it has long been known that lesions of specific afferents result in a decrease in Na⁺-dependent transport in target areas. For example, lesions of the cortical projections to the striatum result in decreased uptake in striatal synaptosomes. These types of studies suggested that there was significant transport into presynaptic terminals, but more recent studies have suggested that these lesions reduce expression of the glial transporters.

Evidence from several complementary strategies strongly suggests that GLT-1. mediates the bulk of NA⁺-dependent transport of EAAs in the CNS. For example, the pharmacological properties of GLT-1 parallel the predominant component of activity observed in rat brain membranes. Based on the enrichment required to purify GLT-1 to homogeneity, it is thought that GLT-1 represents approximately 1% of total brain protein. Selective immunoprecipitation of GLT-1 from solubilized forebrain tissue and reconstitution of the remaining protein in liposomes, suggests that GLT-1 mediates 90% of transport activity. Anti-sense knock-down of GLT-1 results in the dramatic reductions in synaptosomal transporter activity in several forebrain region. Synaptosomal uptake in mice genetically deleted of GLT-1 is 5% of normal. Finally, electrophysiological recording of transporter mediated currents in brain preparations strongly suggest that GLT-1 has a primary role for the clearance of glutamate during synaptic transmission in several forebrain regions.

The expression of GLT-1/EAAT2 is dynamically regulated both *in vivo* and in vitro. Although GLT-1 is the predominant transporter in the adult CNS, expression is rather low early in development and increases during synaptogenesis in both rats and humans. As described above, lesions of projections to a particular target nucleus results in decreased expression of both glial transporters, GLT-1 and GLAST. These data suggest that the presence of neurons induces and/or maintains expression of the glial transporters.

Several different groups have demonstrated decreased expression of GLT-1 and/or GLAST in animal models of acute insults to the CNS, including stroke and traumatic brain injury. A loss in GLT-1 expression has been demonstrated in patients with ALS. Furthermore, there is evidence of decreased expression of these transporters in humans with chronic neurodegenerative diseases, including Alzheimer's Disease, and Huntington's Disease. Loss of GLT-1 is also a feature of the fatal brain tumor, glioblastoma multiforma.

Amyotrophic lateral sclerosis (ALS) is the most common form of adult motor neuron disease in which there is progressive degeneration of both the upper motor neurons in the cortex and the lower motor neurons in the brain stem and spinal cord. The majority of ALS cases (95%) are apparently sporadic (SALS), while approximately 5% are familial (FALS). Cleveland DW, Rothstein JD., Nat.Rev.Neurosci. (2001); 2:806-819; Kuncl RW, Crawford TO, Rothstein JD, Drachman DB. Motor neuron diseases. In: Asbury AK et al., editors. Diseases of the Nervous System. 2 ed. Philadelphia: W.B. Saunders, 1992:1179-1208. FALS cases were found to be associated with mutations in SOD-1, (Andersen PM et al. Genetics of amyotrophic lateral sclerosis: an overview. In: Brown RH, Jr., Meininger V, Swash M, editors. Amyotrophic lateral sclerosis. London: Martin Dunitz, 2000:223-250; Brown RH, Jr. Amyotrophic lateral sclerosis. Insights from genetics. Arch Neurol 1997; 54:1246-1250; Cleveland DW, Rothstein JD. Nat.Rev.Neurosci. 2001; 2:806-819) the gene that encodes copper-zinc superoxide dismutase (CuZnSOD). SOD1 mutations account for about 15-20% of all FALS. SOD1 mutations have been used to generate transgenic mouse models; G93A, G37R, G86R and G85R SOD1 all produce reliable motor neuron degeneration in transgenic mice over-expressing the mutant protein. Cleveland DW., Neuron 1999; 24:515-520; Cleveland DW et al., Nature 1995; 378:342-343; Cleveland DW, Rothstein JD. Nat.Rev.Neurosci. 2001; 2:806-819. Pathogenic events "downstream" of mutant SOD1 toxicity include excitotoxicity, neuroinflammation and apoptosis. Increasingly, these downstream events have been the target of pharmacotherapy- in some cases successfully altering disease course. Multiple other genes or chromosomal localization have been identified in other familial variants of ALS.

Common to both familial and sporadic ALS is the loss of the astroglial glutamate transporter EAAT2 protein. As described above, the astroglial transporter EAAT2 is the predominant protein responsible for the bulk of synaptic clearance of glutamate. In particular, EAAT2 protects against excitotoxic neurodegeneration. Evidence of abnormalities in glutamate handling initially arose in ALS from discovery of large increases in cerebral fluid levels of glutamate in ALS patients, findings now reported in ∼40% of sporadic ALS patients. Rothstein JD et al. Ann.Neurol. 1990; 28:18-25; Spreux-Varoquaux O et al. JNeurol Sci. 2002; 193:73-78. Measurement of functional glutamate transport in ALS tissue revealed a marked diminution in the affected ALS brain regions. The loss of functional glutamate transporter is likely the result of a dramatic loss of astroglial glutamate transporter protein EAAT2, which can be in up to 65% of sporadic ALS patients. Rothstein JD et al., Ann.Neurol. 1994; 36:282; Rothstein JD, et al. N Engl. J Med 1992; 326:1464-1468; Rothstein JD et al., Ann Neurol 1995; 38:73-84. Regardless of the mechanism, lowering EAAT2 with antisense oligonucleotides has demonstrated that loss of transport activity directly provokes neuronal death. Furthermore, expression of at least three (G85R, G93A, G37R) SOD1 mutant in transgenic mice-all lead to a loss of the EAAT2 protein and its function. Rothstein JD et al., Neuron 1996; 16:675-686; Rothstein JD et al., Proc Natl Acad Sci USA 1993; 90:6591-6595. In aggregate, these and other studies studies suggest that that the functional loss of EAAT2 (associated with astrocyte dysfunction), contributes to the loss of motor neurons in both inherited and sporadic ALS. Recently, we also documented a loss of the GLT-1/EAAT2 protein in a new rat transgenic model of the disease. Howland DS et al., Proc.Natl.Acad.Sci. USA 2002; 99:1604-1609. *Notably, loss of transporter protein precedes actual degeneration of motor neurons and their axons in the rat model.*

Two labs recently provided important data as to the importance of EAAT2 as a therapeutic. Dr. Glen Lin, reported that a 2 fold overexpression of EAAT2, in transgenic mice, leads to neuroprotection in vitro, and delayed onset of disease in ALS mice. Guo, H. et al. Hum Mol Genet. 2003; 12:2519-2532. Similarly, Dr. Margaret Sutherland, has reported that a five fold over expression of EAAT2 in transgenic mice, can increase survival of G93A SOD1 mice by at least 30 days (and in several animals many months longer). Maguire JL, et al. Soc Neurosci Abstr. 2001; 27:607.9; Sutherland M.L., et al. Soc Neurosci Abs. 2003. In addition, her lab has reported that increased EAAT2 can also attenuate seizures and significantly diminished both seizures and tumor growth in glioma xenografted rodent. As will be shown below, we have also generated, data that suggest that over expression of EAAT2 can delay disease onset in ALS mice, using novel therapeutics.

Even though GLT-1 expression- is extremely high *in vivo,* 'normal' astrocytes maintained in culture express essentially no detectable mRNA or protein. Co-culturing astrocytes with neurons induces glial expression of GLT-1, suggesting that neurons induce and/or maintain expression of GLT-1 *in vitro.* This effect of neurons is, at least in part, mediated by a soluble secreted molecule. Several small molecules mimic this, effect of neurons, including dbcAMP, epidermal growth factor, pituitary adenylate cyclase-activating peptide, and immunophilin. In all of these cases the increases in GLT-1 protein expression are accompanied by an increase in GLT-1 mRNA and a change in the morphology of the astrocytes that many believe are reminiscent of differentiation.

The effects of dbcAMP are blocked by an inhibitor of protein kinase A. It has been shown that the increase in GLT-1 expression induced by dbcAMP, epidermal growth factor, or neuron conditioned medium are all blocked by an inhibitor of either phosphatidylinositol 3-kinase or an inhibitor of the transcription factor NF-κB. Otherwise, little is known about the mechanisms that actually control GLT-1 expression. Thus, the identification-of the EAAT2 promoter provides a valuable tool to understand EAAT2 regulation and to develop assays to control its synthesis.

As used herein, the term "EAAT2" refers to the human astroglial glutamate transporter 2 gene. See, e.g., U.S. Patent No. 5,658,782 which discloses the human EAAT22 cDNA sequence, the disclosure of the which is specifically incorporated herein by reference. As used herein, the term "GLT-1" refers to the rodent astroglial glutamate transporter 2 gene.

As used herein, the term "promoter" generally refers a region of genomic DNA, usually found 5' to an mRNA transcription start site. Promoters are involved in regulating the timing and level of mRNA transcription and contain, for example, binding sites for cellular proteins such as RNA polymerase and other transcription factors. As used interchangeably herein, the terms "EAAT2 promoter", "EAAT2 promoter region" and the like include the region of genomic DNA found 5' to the EAAT2 mRNA transcription start site. In preferred embodiments, the EAAT2 promoter comprises SEQ ID NO:1, 2, 3, or 4, or fragments thereof When inserted into a promoterless reporter construct, preferred EAAT2 promoter fragments are able to direct transcription of the reporter gene.

In one embodiment, the EAAT2 promoter includes SEQ ID NO:1 (e.g., nucleotides 1-4696 of SEQ ID NO:1). In another embodiment the EAAT2 promoter includes a P1 region, which comprises nucleotides 733-3450 of SEQ ID NO:1 (also set forth as SEQ ID NO:2). In another embodiment, the EAAT2 promoter includes a P2 region, which comprises nucleotides 733-3186 of SEQ ID NO:1 (also set forth as SEQ ID NO:3). In still another embodiment, the EAAT2 promoter includes a P3 region, which comprises nucleotides 2590-3450 of SEQ ID NO:1 (also set forth as SEQ ID NO:4).

The EAAT2 promoter activation molecules of the present invention provide therapeutic agents for neurological and psychiatric disorders. As used herein, the term 'neurological disorder' includes a disorder, disease or condition which affects the nervous system, e.g., the central nervous system. The neurological disorders that can be treated in accord with the present invention include specific disorders that have been reported to be associated with excitotoxicity. Particularly included are specified neurological disorders affecting motor neuron function. Neurological disorders include, but are not limited to, amyotrophic lateral sclerosis (ALS), trinucleotide repeat expansion disorders (e.g.; Huntington's disease (HD), spinal and bulbar muscular atrophy, spinocerebellar ataxia types 1,2,6, and 7, dentatorubropallidoluysian atrophy, and Machado-Joseph disease), α-synucleinopathies (e.g., Parkinson's disease (PD), dementia with Lewy bodies (DLB), and multiple system atrophy (MSA)), multiple sclerosis (MS), Alzheimer's disease, brain tumors (e.g., glioblastoma), stroke/ischemia, cerebrovascular disease, epilepsy. (e.g., temporal lobe epilepsy), HIV-associated dementia, Korsakoff's disease, chronic pain, neurogenic pain, painful neuropathies, headaches (e.g., migraine headaches), Pick's disease, progressive supranuclear palsy, Creutzfeldt-Jakob disease, Bell's Palsy, aphasia, sleep disorders, glaucoma, and Meniere's disease.

In addition, the EAAT2 promoter activation molecules of the present invention provide therapeutic agents for modulation of normal glutamate neurotransmission associated with brain functions such as learning and memory. The molecules described herein can be administered to a subject in need of such treatment for the enhancement of memory and learning.

As used herein, the term 'psychiatric disorder' refers diseases and disorders of the mind, and includes diseases and disorders listed in the Diagnostic and Statistical Manual of Mental Disorders - Fourth Edition (DSM-IV), published by the American Psychiatric Association, Washington D.C. (1994). Psychiatric disorders-include, but are not limited to, anxiety disorders (e.g., acute stress disorder agoraphobia, generalized anxiety disorder, obsessive-compulsive disorder, panic disorder, posttraumatic stress disorder, separation anxiety disorder, social phobia, and specific phobia), childhood disorders, (e.g., attention-deficit/hyperactivity disorder, conduct disorders, and oppositional defiant disorder); eating disorders (e.g., anorexia nervosa and bulimia nervosa), mood disorders (e.g., depression, bipolar disorder, cyclothymic disorder, dysthymic disorder, and major depressive disorder), personality disorder (e.g., antisocial personality disorder, avoidant personality disorder, borderline personality disorder, dependent personality disorder, histrionic personality disorder, narcissistic personality disorder, obsessive-compulsive personality disorder, paranoid personality disorder, schizoid personality disorder, and schizotypal personality disorder), psychotic disorders (e.g., brief psychotic disorder, delusional disorder, schizoaffective disorder, schizophreniform disorder, schizophrenia, and shared psychotic disorder), substance-related disorders (e.g., alcohol dependence, amphetamine dependence, cannabis dependence, cocaine dependence, hallucinogen dependence, inhalant dependence, nicotine dependence, opioid dependence, phencyclidine dependence, and sedative dependence), adjustment disorder, autism, delirium, dementia, multi-infarct dementia, learning and memory disorders (e.g., amnesia and age-related memory loss), and Tourette's disorder.

As noted, neurological and psychiatric disorders of specific interest include those associated with abnormal release or removal of excitotoxic amino acids such as glutamate. Several CNS neuron types are especially adversely affected by excitotoxic glutamate. See e.g., Choi, D.W. (1988) Neuron 1: 623; and references cited therein. Specifically preferred neurological disorders include AD, HD, PD with ALS being especially preferred.

### III. Screening Assays

The invention provides a method (also referred to herein as a "screening assay") for identifying modulators, i.e., candidate or test compounds or agents (e.g., nucleic acids, peptides, peptidomimetics, small molecules, or other drugs) which bind to the EAAT2 promoter, and/or which have a stimulatory or inhibitory effect on, for example, EAAT2 promoter activity.

In one embodiment, the invention provides assays for screening candidate or test compounds which are modulators EAAT2 promoter activity. In another embodiment, the invention provides assays for screening candidate or test compounds which bind to or modulate the activity of an EAAT2 promoter. The test compounds of the present invention can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection. The biological library approach is limited to peptide libraries, while the other four approaches are applicable to peptide, non-peptide oligomer or small molecule libraries of compound (Lam, K.S. (1997) Anticancer Drug Des. 12:45).

Examples of methods for the synthesis of molecular libraries can be found in the art, for example, in: DeWitt et al. (1993) Proc. Natl. Acad. USA 90:6909; Erb et al. (1994) Proc. Natl. Acad. Sci. USA 91:11422; Zuckermann et al. (1994). J. Med. Chem. 37:2678; Cho metal. (1993) Science 261:1303; Carrell et al. (1994) Angew. Chem. Int. Ed. Engl. 33:2059; Carell et al. (1994) Angew. Chem. Int. Ed. Engl. 33:2061; and Gallop et al. (1994) J. Med. Chem. 37:1233.

Libraries of compounds may be presented in solution (e.g., Houghten (992) Biotechniques 13:412-421), or on beads (Lam (1991) Nature 354:82-84), chips (Fodor (1993) Nature 364:555-556), bacteria (Ladner U.S. Pat. No. 5,223,409), spores (Ladner Us. Pat. No. '409), plasmids (Cull et al. (1992) Proc. Natl. Acad. Sci. USA 89:1865-1869) or on phage (Scott and Smith (1990) Science 249:386-390); (Devlin (1990) Science 249:404-406); (Cwirla et at. (1990) Proc. Natl. Acad. Sci. USA 87:6378-6382); (Felici (1991) J. Mol. Biol. 222:301-310); (Ladner supra.).

In a preferred embodiment, an assay is a cell-based assay in which a cell which expresses a reporter gene operatively linked to an EAAT2 promoter or portion thereof (e.g., whose expression is under the control of the EAAT2 promoter or portion thereof) is contacted with a test compound and the ability of the test compound to modulate EAAT2 promoter activity is determined. Determining the ability of the test compound to modulate EAAT2 promoter activity can be accomplished by monitoring reporter gene expression (e.g., reporter mRNA or polypeptide,expression level) or activity, for example. As described elsewhere herein, the reporter can be any detectable marker. For example, the reporter can be a nucleic acid sequence, the expression of which can be measured by, for example, Northern blotting, RT-PCR, prime extension, or nuclease protection assays. The reporter may also be a nucleic acid sequence that encodes a polypeptide, the expression of which can be measured by, for example, Western blotting, ELISA, or RIA assays. Reporter expression can also be monitored by measuring the activity of the polypeptide encoded by the reporter using, for example, a standard glutamate transport assay, a luciferase assay, a β-galactosidase assay, a chloramphenicol acetyl transferase (CAT) assay, or a fluorescent protein assay.

The level of expression or activity of a reporter under the control of the EAAT2 promoter in the presence of the candidate compound is compared to the level of expression or activity of the reporter in the absence of the candidate compound. The candidate compound can then be identified as a modulator of EAAT2 promoter activity based on this comparison. For example, when expression of reporter mRNA or protein expression or activity is greater (statistically significantly greater) in the presence of the candidate compound than in its absence, the candidate compound is identified as a stimulator of EAAT2 promoter activity. Alternatively, when expression or activity of reporter mRNA or protein is less (statistically significantly less) in the presence of the candidate compound than in its absence, the candidate compound is identified as an inhibitor of EAAT2 promoter activity.

The ability of the test compound to bind to the EAAT2 promoter and/or to modulate the binding of proteins (e.g., transcription factors) to the EAAT2 promoter can also be determined. Determining the ability of the test compound to bind to and/or modulate EAAT2 promoter binding to a binding protein can be accomplished, for example, by coupling the test compound, the EAAT2 promoter or the binding protein with a radioisotope or enzymatic label such that binding of the EAAT2 promoter to the test compound or the binding protein can be determined by detecting the labeled component in a complex. For example, compounds (e.g., the test compound, the EAAT2 promoter, or a binding protein) can be labeled with ³²P, ¹²⁵I, ³⁵S, ¹⁴C, or ³H, either directly or indirectly, and the radioisotope detected by direct counting of radioemission or by scintillation counting. Alternatively, compounds can be enzymatically labeled with, for example, horseradish peroxidase, alkaline phosphatase, or luciferase, and the enzymatic label detected by determination of conversion of an appropriate substrate to product.

It is also within the scope of this invention to determine the ability of a compound (e.g., a test compound or EAAT2 promoter binding protein) to interact with the EAAT2 promoter without the labeling of any of the interactants. For example, a microphysiometer can be used to detect the interaction of a compound with the EAAT2 promoter without the labeling of either the compound or the EAAT2 promoter (McConnell, H. M. et al. (1992) Science 257:1906-1912). As used herein, a "microphysiometer" (e.g., Cytosensor) is an analytical instrument that measures the rate at which a cell acidifies its environment using a light-addressable potentiometric sensor (LAPS). Charges in this acidification rate can be used as an indicator of the interaction between a compound and the EAAT2 promoter.

In another embodiment, the assay is a cell-free assay in which an EAAT2 promoter or portion thereof is contacted with a test compound and the ability of the test compound to modulate (e.g., stimulate or inhibit) the activity of the EAT2 promoter or portion thereof is determined. Determining the ability of the test compound to modulate the activity of an EAAT2 promoter can be accomplished, for example, by determining the ability of the EAAT2 promoter to bind, to an EAAT2 promoter target molecule by one of the methods described above for determining direct binding. Determining the ability of the EAAT2 promoter to bind to an EAAT2 promoter target molecule can also be accomplished using a technology such as real-time Biomolecular Interaction Analysis (BIA). Sjolander, S. and Urbaniczky, C. (1991) Anal. Chem. 63:2338-2345 and Szabo et al. (1995) Curr. Opin. Struct Biol. 5:699-705. As used herein, "BIA" is a technology for studying biospecific interactions in real time, without labeling any of the interactants (e.g., BIAcore). Changes in the optical phenomenon of surface plasmon resonance (SPR) can be used as an indication of real-time reactions between biological molecules.

In yet another embodiment, the cell-free assay involves contacting an EAAT2 promoter or portion thereof with a known compound which binds the EAAT2 promoter (e.g., a component of the basal transcription machinery) to form an assay mixture, contacting the assay mixture with a test compound, and determining the ability of the test compound to interact with the EAAT2 promoter, wherein determining the ability of the test compound to interact with the EAAT2 promoter comprises determining the ability of the EAAT2 promoter to preferentially bind to or modulate the activity of an EAAT2 promoter target molecule.

In more than one embodiment of the above assay methods of the present invention, it may be desirable to immobilize either EAAT2 promoter or its target molecule to facilitate separation of complexed from uncomplexed forms of one or both of the molecules, as well as to accommodate automation of the assay. Binding of a test compound to an EAAT2 promoter, or interaction of an EAAT2 promoter with a substrate or target molecule in the presence and absence of a candidate compound, can be accomplished in any vessel suitable for containing the reactants. Examples of such vessels include microtiter plates, test tubes, and micro-centrifuge tubes. In one embodiment, a fusion protein can be provided which adds a domain that' allows one or both of the proteins to be bound to a matrix. For example, glutathione-S-transferase/target fusion proteins can be adsorbed onto glutathione sepharose beads (Sigma Chemical, St. Louis, Mo.) or glutathione derivatized micrometer plates, which are then combined with the test compound or the test compound and either the non-adsorbed target protein or EAAT2 promoter, and the mixture incubated under conditions conducive to complex formation (e.g., at physiological conditions for salt and pH). Following incubation, the beads or microtiter plate wells are washed to remove any unbound component, the matrix immobilized in the case of beads, complex determined either directly or indirectly, for example, as described above. Alternatively, the complexes can be dissociated from the matrix, and the level of EAAT2, promoter binding or activity determined using standard techniques.

Other techniques for immobilizing proteins or nucleic acids on matrices can also be used in the screening assays of the invention. For example, either an EAAT2 promoter or an EAAT2 promoter substrate or target molecule can be immobilized utilizing conjugation of biotin and streptavidin. Biotinylated EAAT2 promoter, substrates, or target molecules can be prepared from biotin-NHS (N-hydroxysuccinimide) using techniques known in the art (e.g., biotinylation kit, Pierce Chemicals, Rockford, III.), and immobilized in the wells of streptavidin-coated 96 well plates (Pierce Chemical). Alternatively, antibodies reactive with EAAT2 promoter or target molecules but which do not interfere with binding of the EAAT2 promoter to its target molecule can be derivatized to the wells of the plate, and unbound target or EAAT2 promoter trapped in the wells by antibody conjugation. Methods for detecting such complexes, in addition to those described above for the GST-immobilized complexes, include immunodetection of complexes using antibodies reactive with the EAAT2 promoter or target molecule, as well as enzyme-linked assays which rely on detecting an enzymatic activity associated with the EAAT2 promoter or target molecule.

In yet another aspect of the invention, the EAAT2 promoter can be used as "bait" in a one-hybrid assay (see, e.g., BD Matchmaker One-Hybrid System (1995) Clontechniques X(3):2-4; BD Matchmaker library Construction & Screening Kit (2000) Clontechniques XV(4):5-7; BD SMART technology overview (2002) Clontechniques XVII(1):22-28; Ausubel, F. M., et al. (1998 et seq.) Current Protocols in Molecular Biology Eds. Ausubel, F. M., et al., pp. 13.4.1-13.4.10) to identify proteins which bind to or interact with the AAT2 promoter ("EAAT2 promoter-binding proteins" or "EAAT2 promoter-bp") and are involved in EAAT2 promoter activity. Such EAAT2 promoter-binding proteins are also likely to be involved in the regulation of transcription from the EAAT2 promoter.

In another aspect, the invention pertains to a combination of two or more of the assays described herein. For example, a modulating agent can be identified using a cell-based or a cell-free assay, and the ability of the agent to modulate the activity of an EAAT2 promoter can be confirmed in vivo, e.g., in an animal such as an animal model for a neurological disease.

This invention further pertains to novel agents identified by the above-described screening assays. Accordingly, it is within the scope of this invention to further use an agent identified as described herein in an appropriate animal model (e.g., an animal model for a neurological disease). For example, an agent identified as described herein (e.g_{.}, an EAAT2 promoter modulating agent or an EAAT2 promoter binding protein) can be used in an animal model to determine the efficacy, toxicity, or side effects of treatment with such an agent. Alternatively, an agent identified as described herein can be used in an animal model to determine the mechanism of action of such an agent. Furthermore, this invention pertains to uses of novel agents identified by the above-described screening assays for treatments as described herein.

Agents are described herein that are identified using the screening methods delineated herein. These compounds include a variety of chemical structures as identified herein, including compounds having a β-lactam ring system, more specifically, β-lactam antibiotic compounds, including penicillin class, cephalosporin class, carbapenam class and monobactam class compounds.

### IV. Methods of Treatment

Use of an antibiotic in accordance with the present invention has utility in treating neurological and psychiatric disorders, by administering a therapeutically effective amount of a pharmaceutical composition comprising an antibiotic as an EAST2 promoter modulator to a subject (e.g., a mammal, such as a human).

To modulate EAAT2 promoter activity, and thereby modulate EAAT2 gene expression, e.g.; a compound disclosed herein or identified by the screening assays of the invention, can be administered to a cell or a subject. Administration of an EAAT2 promoter modulator to mammalian, cells (including human cells) can modulate (e.g., up- or down regulate) EAAT2 mRNA and/or polypeptide expression, thereby up- or drown-regulating glutamate transport into the cell. In such methods, the EAAT2 promoter can be administered to a mammal (including a human) by known procedures.

An antibiotic according to the invention (which includes prophylactic use) can be used for manufacture of a medicament suitable for administration of a therapeutically effective amount of an EAAT2 promoter modulator to an animal in need thereof, including a mammal, particularly a human. Such a medicament will be suitably administered to subjects, particularly humans, suffering from, having, susceptible to, or at risk for a neurological disease or disorder.

For therapeutic applications, the medicament manufactured according to the invention may be suitably administered to a subject such as a mammal, particularly a human, alone or as part of a pharmaceutical composition, comprising the EAAT2 modulator together with one or more acceptable carriers thereof and optionally other therapeutic ingredients. The carrier(s) must be "acceptable" in the sense of being compatible with the other ingredients of the formulation and not deleterious to the recipient thereof.

The pharmaceutical compositions of the invention include those suitable for oral, rectal, nasal, topical (including buccal and sublingual), vaginal or parenteral (including subcutaneous, intramuscular, intravenous and intradermal) administration. The formulations may conveniently be presented in unit dosage form, e.g. tablets and sustained release capsules, and in liposomes, and may be prepared by any methods well known in the art of pharmacy. See, for example, Remington's Pharmaceutical Sciences, Mack Publishing Company, Philadelphia, PA (17^{th} ed. 1985).

Such preparative methods include the step of bringing into association with the molecule to be administered ingredients such as the carrier which constitutes one or more accessory ingredients. In general, the compositions are prepared by uniformly and intimately bringing into association the active ingredients with liquid carriers, liposomes or finely divided solid carriers or both, and then if necessary shaping the product.

Compositions of the present invention suitable for oral administration may be presented as discrete units such as capsules, sachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion, or packed in liposomes and as a bolus, etc.

A tablet may be made by compression or molding, optionally with one or more accessary ingredients. Compressed tablets may be prepared by compressing in a suitable machine the active ingredient in a free-flowing form such as a powder or granules, optionally mixed with a binder, lubricant, inert diluent, preservative, surface-active or dispersing agent. Molded tablets may be made by molding in a suitable machine a mixture of the powdered compound moistened with an inert liquid diluent. The tablets optionally may be coated or scored and may be formulated so as to provide slow or controlled release of the active ingredient therein.

Compositions suitable for topical administration include lozenges comprising the ingredients in a flavored basis, usually sucrose and acacia or tragacanth; and pastilles comprising the active ingredient in an inert basis such as gelatin and glycerin, or sucrose and acacia.

Compositions suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile, suspensions which may include suspending agents and thickening agents. The formulations may be presented in unit-dose or multi-dose containers, for example, sealed ampules and vials, and may be stored in a freeze dried (lyophilized) condition requiring only the addition of the sterile liquid carrier, for example water for injections, immediately prior to use. Extemporaneous injection solutions and suspensions may be prepared from sterile powder, granules and tablets.

Application of the subject therapeutics often will be local, so as to be administered at the site of interest. Various techniques can be used for providing the subject compositions at the site of interest, such as injection, use of catheters, trocars, projectiles, pluronic gel, stents, sustained drug release polymers or other device which provides for internal access Where an organ or tissue is accessible because of removal from the patient, such organ or tissue may be bathed in a medium containing the subject compositions, the subject compositions may be painted onto the organ, or may be applied in any convenient way.

It will be appreciated that actual preferred amounts of a given EAAT2 modulator of the invention used in a given therapy will vary to the particular active compound being utilized, the particular compositions formulated, the mode of application, the particular site of administration, the patient's weight, general health, sex, etc., the particular indication being treated, etc. and other such factors that are recognized by those skilled in the art including the attendant physician or veterinarian. Optimal administration rates for a given protocol of administration can be readily determine by those skilled in the art using conventional dosage determination tests.

The invention will be further described in the following examples. It should be understood that these examples are for illustrative purposes only and are not to be construed as limiting this invention in any manner.

### EXAMPLES

### EXAMPLE 1: In vitro analysis of EAAT2 protein expression.

**Screening Assay for EAAT2 Protein overexpression.** Spinal cord organotypic cultures and astroglial cultures are used to screen for drugs capable of stimulating EAAT2 syntheses and function. Organotypic cultures offer the advantage in that they maintain the normal architecture of neuron-astroglial interactions in vitro and are derived from post natal tissue; thus may better reflect astroglial responses in vivo (rather than embryonic cells). Thus a drug that acts either on an astrocyte - or induces neurons to secrete factors that alert astrocytes - better reflects the "natural" condition of delivering a drug to a whole animal.

**Bioassay Method:** Figure 1 depicts the results from a screen of 1040 FDA approved drugs. The screen revealed that β-lactam-antibiotics serve as inducers of EAAT2 protein. Briefly, the screen includes incubating spinal cord cultures with compounds for 3 days (Figure 1A). Figure 1B depicts a sample slot blot from tissue homogenates to validate the increased EAAT2 expression seen with increasing controls known to occur with dibutyryl cyclic AMP, GDNF or the neuroimmunophilin GPI-1046. All three compounds (dbcAMP, GDNF and GPI-1046) induced a large increase in EAAT2 expression, after three days in culture. Figure 1C depicts a typical data slot blot of EAAT2 protein from an early screen of various, agents. Every blot included control-untreated tissue and a positive control from panel B. Figure 1D depicts screening results from the NIH-NINDS Custom Collection screen of 1040 FDA approved compound. Height of the bars reflects increased EAAT2 protein expression relative to untreated controls. Each blot included at least one untreated control and one known positive control. Figure 1E depicts β-Lactam antibiotics were highly represented in the most potent compounds. These compounds were able to increase EAAT2 protein expression up to 7 fold compared to untreated control cultures, after a 7 days chronic treatment^{.} Figure 1F depicts a dose response analysis for ceftriaxone, revealing EC₅₀ 3.5µM for EAAT2 expression.

**Organotypic Spinal Cord**. Spinal cord organotypic cultures have been described by us in detail in the past. Rothstein JD et al., N. Engl. J. Med. 1992; 326:1464-1468. Briefly, 300 µm sections of rat lumbar spinal cord, from postnatal day 8-10 rat pups, are placed on Millipore Millicell CM semipermeable membranes. Each well contains 5 slices (**Figure 1A**)**.** Fifty-100 cultures can be prepared weekly. Each drug (10-100 µM) was added for 3 days, along with cell culture medium/serum. Cultures were harvested and 5-50 µg of tissue was applied to slot blot apparatus for detection of **EAAT2** by standard Western blotting/chemiluminescence methods described in the past. Kuncl RW et al., Motor neuron diseases. In: Asbury AK et al., editors. Diseases of the Nervous System. 2 ed. Philadelphia: W.B. Saunders, 1992:1179-1208; Rothstein JD et al., Neuron 1994; 13:713-725. All antipeptide antibodies were affinity purified and highly specific for transporter subtypes. A typical slot-blot analysis, is shown in **Figure 1B**, 1C. By this method, we can reliably detect increases greater than 50% of expressed protein. For each antibody slot blot, the homogenates used are expected to be within the linear range for antibody detection, based on prior standard curves.

**Screening Library-Assay Design**. The library of compound for these first studies was the NINDS Custom Collection from Microsource Discovery. The library is composed of 1040 compounds in 96 well plates, that also included positive control for transporter synthesis (dibutyryl cyclic AMP. [dbcAMP], GDNF). The library is a unique collection of known bioactive compounds that permit the simultaneous evaluation of hundreds of marketed drugs and biochemical standards. Each compound was studied at a final concentration of 10-100µM. All assays were performed in duplicate. Atypical slot blot is shown in **Figure 1C**.

**Data Analysis**. All blots were analyzed by laser densitometry (BioRad Image Quant) and the duplicate points were averaged. The complete result dataset from the 1040 compounds is shown in **Figure 1D**. Each blot included a positive control standard (e.g. dbcAMP) and a negative control standard (e.g. serum DMSO). Data was kept in Excel, Spreadsheets, using a numerical/text coding system. All positive drugs (positive defined as at least a 50% increased in protein expression) were re-evaluated.

**RESULTS: Screened, Drugs Can Increase EAAT2 In vitro**. After screening 1040 compounds, we were able to identify more than 10 related compounds capable of increasing EAAT2 protein levels by 3.5 to 7 fold (**see** **Figure 1E**). In total, we identified 80 compounds capable of increasing EAAT2 by 2 fold or more in the first screen. Of that list, β-lactam, antibiotics were overly represented and were the most common structural motif observed in all compounds fifteen different beta lactam antibiotics were active. As shown in **Figure 1E**, these β-lactams were all capable of increased EAAT2 protein expression. A follow-up dose response analysis (**Figure 1F**) revealed and EC₅₀ for protein expression for ceftriaxone of 3.5 µM.

### Example 2: EAAT2 promoter reporter activation. Veneration of COS7 and Human Astroglial Promoter reporter cell lines.

**EAAT2 promoter (E2P) isolation and Reporter Generation**. A 2.7 kb EAAT2 promoter fragment was obtained by cutting the PAC clone RP4-683L5 with Kpn1 and Nco1. Previous studies document that sequence, 5' of EAAt2 coding region, has promoter motifs and can be activated in vitro. The promoter was cloned into the pGL3-basic luciferase reporter vector (Promega) (refered to as pE2P-GL3) or pEGFP-1 plasmid (Clontech) (named pE2P-eGFP). E2P was also cloned into a pLck-eGFP plasmid (a myristoylated version of eGFP that targets the eGFP protein to the membrane). Finally, E2P was also cloned into a pE2P-Luciferase-IRES-Lck-eGFP plasmid, which has the fragment E2P-Luciferase from pE2P-GL3, followed by an IRES (internal ribosomal entry site), followed by Lck-eGFP (named pELILE). In this last constructs E2P drives the expression of both Luciferase and eGFP at the same time.

**Generation of E2P-eGFP and Bac-EAAT1-eGFP Transgenic mice (****Figure 2****)**. To provide screening cell lines for the assays we have now successfully generated two transgenic mice that express the EAAT2 promoter fragments (E2P) or the full length EAAT1 promoter (Bac-EAAT1). As shown in **Figure 2** we have generated E2P transgenic mice that demonstrate widespread expression of the EAAT2 promoter reporter in the CNS. Similarly we generated Bac-EAAtl mice and expressing cells. Recently the Heintz group also generated an EAAT1 Bac-reporter based mouse based on a similar Bac construct used in our own mice.

**Screening assays.** Lipofectamine 2000 reagent was used to transect, Cos-7 and HEK-293 cells. Human cortical astroglial cell were obtained from our collegue, Dr. Avi Nath. The EpE2P-eGFP, pE2P-Lck-eGFP, and pELILE contain a Neomycin resistance gene that permitted establishment of stable cell lines. For the human astroglial cells, SV40 was used to immortalize the cell. Stably transfected cells were seeded on 24-well plates, incubated with 10 µM compound solution for 48 hours and the fluorescence intensity was recorded with an automated reader (SpectraGeminiXS).

**RESULTS: Identification of EAAT2 Promoter activating compounds (****Figure 3****).** From the original NINDS screen, we identified numerous β lactam compounds capable of potently activating EAAt 2 promoter. As shown in **Figure 3****,** most β-lactams were able to increase EAAT promoter far more than the known positive control, dibutyrl cyclic AMP. All compounds were active at a pharmacologically relevant concentration of 1-10µM- a concentration range that these compounds can be found in the CNS after standard anti-bacterial therapy (e.g. ceftriaxone).

### Example 3: In vivo activation of EAAT expression / function.

In vivo activation of protein expression/function can be assessed as delineated in the example below using ceftriaxone as the test compound.

### Ceftriaxone Increases Brain GLT1 level (Figure 4).

To determine if a drug identified in Phases 1 and 2 could actually induce EAAT expression in vivo, we administered ceftriaxone to rats (n=5) (and mice, n=3) daily. Ceftriaxone was administered at a dose known to lead to CNS levels, 200 mg/kg ip. After 5 days of chronic daily administration animals were sacrificed and brain tissue harvested. As shown in **Figure 4A****,** **4B****,** ceftriaxone therapy led to 3 fold increase in brain GLT1 levels, as well as its normal splice product, GLT1b. This increase is comparably to the promoter activation results seen in vitro (**Figure 3**) . Western blots for the astroglial glutamate transporter GLAST as well as the two neuronal glutamate transporters, EAACI and EAAT4, showed no alteration in transporter expression after ceftriaxone therapy **(****Figure 4C**, **4D**) . Similarly, the constitutive protein, actin, was unchanged by ceftriaxone administration **(****Figure 4A**, **4C**).

### Example 4: Neuroprotection of compounds.

To evaluate the potential neuroprotection afforded by increased expression of EAAT2 by promoter activating drugs, we have conducted several in vitro and in vivo experiments where glutamate toxicity contributes to neuronal death.
Neuroprotection can be assessed as delineated in the example below using β-lactam antibiotics as the test compound.

**In Vitro Model of Ischemia - Oxygen glucose deprivation (****Figure 6A****)** The in vitro model of oxygen glucose deprivation (OGD) is a well known and well accepted model of acute neural injury. In our in-vitro model of ischemia, one hour of oxygen glucose deprivation (OGD) is lethal to cultured neurons, with toxicity known to involve excess glutamate. However, when these cultures are preconditioned 24 hours prior to the lethal condition with transient OGD (5 minutes), there is a dramatic and robust resistance of neurons to cell death. The data indicate that this neuroprotection may be due, in part, to increased expression of GLT1.

**Method.** Primary cortical mixed neuronal-glial cell cultures are prepared from gestation day 14-16 CD1 mice. The preparation of these cultures from mouse fetal cortex is well-described. Experiments are performed at days in vitro 13-15. In the experimental condition, cultures are subjected to oxygen glucose deprivation (OGD), an in-vitro model of ischemia. Cortical cells are either subjected to control treatment (media, modified Earle's balanced salt solution including glucose and bubbled with 5% CO₂ 95% O₂, is changed alongside treatment groups, but no OGD is performed), or 5 minutes (sublethal) of OGD (using modified Earless balanced salt solution which is devoid of glucose and bubbled with 10%H₂, 85%N₂, and 5% to deoxygenate) . Anaerobic conditions are achieved using an anaerobic chamber at 37°C. OGD is terminated by exchange of media back to oxygenated growing medium. Twenty-four hours following the above, cortical cells are subjected either to no treatment, or one hour of OGD. neuronal survival is determined by computer-assisted cell counting after staining with the fluorescent vital dyes propidium iodide (as an indicator of neuronal death) and Hoechst 33342 (as an indicator of total number of neurons) and is presented as percent of cell death. Glial nuclei fluoresce at a lower intensity and are gated out. Drugs are added (Ceftriaxone 1 µM) 24 hours prior to the first experimental condition, and thus have been in the culture medium 48 hours prior to onset of 1 hour OGD. Following 1 hour OGD, cells are returned to growing medium without drugs.

***Ceftriaxone Neuroprotection.*** Baseline neuronal death in the cultures its 14%, as shown in the no treatment column (NT) of Figure 6A. Data are presented as average neuronal death in separate wells of one experiment. 1 µM Ceftriaxone, when added for 48 hours in these cultures, does not increase the vaseline cell death (NT + Ceftriaxone). When cultures are subjected to 1 hour OGD, neuronal cell death, as expected increases dramatically to approximately 50%. When cultures are preconditioned with 5 minutes of OGD 24 hours prior to 1hour OGD, percent cell death is comparable to no treatment condition, indicating ischemic tolerance of neurons in this condition. This is the well known phenomenon of ischemic tolerances. Importantly, 1 µM Ceftriaxone, when added 48 hours prior.to 1 hour OGD, also protects neurons from cell death, reducing the percentage of neuronal cell death from 50% to 20%. (similar to ischemic tolerance neuroprotection). Thus, ceftriaxone pretreatment appears to prevent neuronal death in ischemic tolerance.

**In vitro model of chronic motor neurodegeneration (****Figure 6B****)** . A model of chronic neurodegeneration was used, based on the blockade of glutamate transport in spinal cord organotypic cultures, with the non specific inhibitor threo-hydroxyaspartate (THA) or TBOA. Chronic incubation of cultures with THA (or TBOA) leads to chronic increase in extra cellular glutamate and subsequent slow death of motor neurons (over 4 weeks). The organotypic spinal cord culture model was developed to study aspects of glutamate-mediated toxicity (and therapy). It has been useful in pre-clinical drug identification (including riluzole, the only FDA approved drug for ALS, and more recently, celecoxib). Increased expression of glutamate transporter GLT1, by genetic over expression (e.g. transfection or transgenic over expression), in this system, can prevent motor neuron death (not shown) and neuronal death in transgenic animals. Guo H., et al. Hum Mol Genet. 2003; 12: 2519-2532.

To determine if drug induced GLT1 promoter activation, and the subsequent over expression of GLT1 protein could be neuroprotective, we used the organotypic spinal cord paradigm. Organotypic spinal cord cultures were prepared from lumbar spinal cords of 8-day-old rat pups, as described previously. Rothstein JD, et al. Proc Natl Acad Sci U S A. 1993; 90:6591-6595: Ceftriaxone was added with media changes. No drugs were added for the first 7 days following culture preparation. THA was then added to experimental cultures at a concentration of 100µM, which produces death of motor neurons within 3 to 4 weeks. Various concentrations of ceftriaxone-were added as indicated, to achieve final concentrations from 0 to 100 µM. Experiments were always performed with control spinal cord cultures (i.e.; no drugs added), THA alone, ceftriaxone alone, and ceftriaxone + THA.
Experiments at each concentration of ceftriaxone were repeated 3-5 times. The medium, with THA and ceftriaxone at the indicated concentrations, was changed twice a week. After 4 weeks, cultures were fixed, and immuostained for neurofilament (SMI-32, Sternberger) to quantify large ventral horn motor neurons (a well established method to follow motor neuron survival in this systems

**Neuroprotection by ceftriaxone.** As shown in Figure 6B, ceftriaxone treatment prevented motor neurons loss in a dose dependent manner. As shown in Preliminary Data Phases 1 and 2, this concentration of ceftriaxone increases GLT1 protein and function by at least 3 fold. Importantly, the concentrations used in these studies are within the range attainable with oral/parenteral administration of ceftriaxone (1-4 grams/day). Notably, neuroprotection cannot be seen in cultures prepared from GLT-1 null mice (not shown).

### In Vivo Neuroprotection Effect of ceftriaxone on onset and progression of motor neuron disease in the G93A SOD1 Mouse. (Figure 6C, 6D)

To determine if ceftriaxone could alter neurodegeneration in a disease model that involves altered expression of glutamate transporters.we treated G93A SOD1 mice with ceftriaxone. Numerous studies have documented a contributory role for excess glutamate in this mouse model and a role for modulating glutamate receptors or transporters in neuroprotective strategies. Guo H., et al. Hum Mol Genet. 2003; 12: 2519-2532. Modest rover expression by a transgenic approach can alter disease onset and/or survival. Furthermore, recent studies suggest that late administration of drugs, e.g. at time of disease onset, may be more therapeutically relevant.

**Treatment paradigm.** G93A SOD1 mice [(B6.Cg-Tg(SOD1-G93A)lGur/J, high expresser] were treated with ceftriaxone (200 mg/kg ip) starting at approximately 12 weeks of age. Drug treated animals (n=20) and saline injected controls (n=20) were monitored daily for survival and weekly for grip strength (Columbus Instruments) and for body weight, as described previously.

### Ceftriaxone Delays Loss of Grip Strength and Increases Survival

As shown in Figure 7C, ceftriaxone treatment significantly delayed loss or muscle strength. This effect was observed within 7 days after treatment, and persisted for 4 weeks. By 18 weeks of age the strength preservation was lost. In a similar manner, the drug also increased over all survival of the mice by about 7-10 days (Figure 6D). Although this effect is relatively small, the drug was given at the time of disease onset, and thus; even a small effect may have clinical significance. The neuroprotection seen in this study is not likely to be due to the normal antibiotic properties of the drug, since mice have no known infections at 12-16 weeks of age, when prominent muscle strength effects were seen.

## Claims

1. Use of an EAAT2 expression promoting agent that is an antibiotic, in the manufacture of a medicament for treating a neurological disease or disorder associated with altered glutamate transmission.

2. Use according to claim 1, wherein the neurological disease or disorder associated with altered glutamate transmission is associated with learning, memory or cognition.

3. Use according to claim 1 wherein the neurological disease or disorder is selected from the group consisting of Parkinson's disease, Huntington's disease, Alzheimer's disease, multiple sclerosis, amyotrophic lateral sclerosis, acute neurological diseases, epilepsy, spinal cord injury, brain trauma, glaucoma, and psychiatric disorders.

4. Use according to claim 3 wherein the neurological disease or disorder is amyotrophic lateral sclerosis.

5. Use according to claim 1, wherein the antibiotic is a compound identified by a screening assay comprising the steps of:
a) contacting a test antibiotic with a nucleic acid molecule comprising a cDNA molecule and having a nucleotide sequence which is at least about 60% identical to the nucleotide sequence of SEQ ID NO:1, 2, 3, or 4, wherein the nucleic acid molecule is capable of directing mRNA expression from a promoterless reporter vector, or a complement thereof, or a cell comprising said nucleic acid molecule; and
b) determining whether mRNA expression or the polypeptide encoded by the cDNA is modulated,
thereby identifying an antibiotic which is an EAAT2 expression promoting agent.

6. Use according to claim 1, wherein the antibiotic is a compound identified by a screening assay comprising the steps of:
a) contacting a cell that expresses EAAT2 with a test antibiotic; and
b) determining whether expression of the EAAT2 in the cell is modulated in the presence of the test antibiotic compared to in the absence of the test antibiotic, thereby identifying an antibiotic which modulates expression of the EAAT2.

7. Use according to any preceding claim, wherein the antibiotic comprises at least one structural element selected from heterocycles comprising at least one ring sulfur atom, tertiary gamines, quaternary ammonium salts, steroids, polyols, polyketides, guanidine, urea, or arsenate.

8. Use according to any preceding claim, wherein the antibiotic increases EAAT2 production by 200% or more relative to non-regulated EAAT2 production.

9. Use according to any preceding claim, wherein the antibiotic increases EAAT2 production by 300% or morse relative to non-regulated production.

10. Use according to any preceding claim, wherein the antibiotic increases EAAT2 production by 400% or more relative to non-regulated production.

11. Use according to any preceding claim, wherein the antibiotic increases EAAT2 production by 600% or more relative to non-regulated production.

12. Use according to claim 1, wherein the extracellular glutamate concentration is reduced by at least about 50% relative to non-regulated concentrations.

13. Use according to claim 12, wherein the extracellular glutamate concentrations is reduced by at least about 75% relative non-regulated concentration.

14. Use according to any of claims 1 to 4, wherein the antibiotic is a β-lactam antibiotic.

15. Use according to any of claims 1 to 4, wherein the antibiotic is a penicillin class, cephalosporin class, carbapenam class or monobactam class β-lactam antibiotic.

16. Use according to any of claims 1 to 3, wherein the antibiotic is a β-lactam antibiotic selected from the group consisting of benzylpenicillin, procaine benzylpenicillin, penicillin V, penicillin V potassium, benzathine penicillin, hetacillin, cloxacillin, carbenicillin, flucloxacillin, ampicillin, ampicillin sodium, amoxicillin, co-amoxiclav, carboxypenicillin, ticarcillin, timentin, tazocin, piperacillin, pivmecillinam, amoxicillin-clavulanate, oxacillin, bacampicillin HCl, nafcillin sodium, cefaclor, cefadroxil, cefadyl, cefalexin, cefamandole, cefazolin, cefditoren, cefepime, cefetamet, cefdinir, cefixime, cefizox, cefotaxime, cefmetazole, cefobid, cefonicid, cefoperazone, cefoperazone sodium, cefotan, cefotetan, cefoxitin, cefpirome, cefpodoxime, cefpodoxime proxetil, cefprozil, cefradine, ceftazidime, ceftibuten, ceftidoren, ceftin, ceftizoxime, ceftriaxone, ceftriaxone sodium, cefuroxime, cefuroxime axetil, cephalexin, cefzil, cephalothin, cephalothin sodium, cephapirin sodium, Aztreonam, Imipenem, Meropenem, Ertapenem and FK-037

17. Use according to claim 16, wherein the β-lactam antibiotic is selected from the group consisting of penicillin V, penicillin V potassium, amoxicillin, ceftriaxone, ceftriaxone sodium, cephapirin sodium, cefoperazone, cefoperazone sodium, cefadroxil, bacampicillin HCl, ampicillin, ampicillin sodium, cephalothin, cephalothin sodium, and nafcillin sodium.

18. Use according to any of claims 1 to 4, wherein the antibiotic is selected from the group consisting of dequalinium chloride, vancomycin hydrochloride, actinospectacin, oxytetracycline, sisomicin sulfate, erythromycin ethylsuccinate, dihydrostreptomycin sulfate, amprolium, alexidine HCl, clindamycin, enoxotone, rifampin, chloroxine, methenamine, and pyrithione zinc.

19. Use according to claim 17, wherein the antibiotic is ceftriaxone or ceftriaxone sodium.

20. Use according to any of claims 1 to 4, wherein the antibiotic is thiamphenicol.

21. Use according to any of claims 1 to 4, wherein the antibiotic is chloramphenicol.

22. Use according to claim 19 wherein the neurological disease or disorder is amyotrophic lateral sclerosis.

23. An *in vitro* method of increasing EAAT2 protein expression comprising the step of contacting a cell with at least one EAAT2 expression promoting agent that is an antibiotic.

## Patentansprüche

1. Eine Verwendung eines EAAT2-Expressionsfördermittels, das ein Antibiotikum ist, bei der Herstellung eines Arzneimittels zur Behandlung einer neurologischen Krankheit oder Erkrankung, die mit geänderter Glutamatübertragung verbunden ist.

2. Verwendung gemäß Anspruch 1, wobei die neurologische Krankheit oder Erkrankung, die mit geänderter Glutamatübertragung verbunden ist, mit Lernen, Gedächtnis oder Wahrnehmung verbunden ist.

3. Verwendung gemäß Anspruch 1, wobei die neurologische Krankheit oder Erkrankung aus der Gruppe, die aus Parkinson, Chorea Huntington, Alzheimer, multipler Sklerose, amyotropher Lateralsklerose, akuten neurologischen Krankheiten, Epilepsie, Rückenmarksverletzung, Hirntrauma, grünem Star und psychiatrischen Erkrankungen besteht, ausgewählt ist.

4. Verwendung gemäß Anspruch 3, wobei die neurologische Krankheit oder Erkrankung amyotrophe Lateralsklerose ist.

5. Verwendung gemäß Anspruch 1, wobei das Antibiotikum eine Verbindung ist, die durch einen Screening-Assay identifiziert wird, welcher die Folgenden Schritte beinhaltet:
a) In-Kontakt-Bringen eines Testantibiotikums mit einem Nukleinsäuremolekül, das ein cDNA-Molekül beinhaltet und eine Nukleotidsequenz, die mindestens zu 60 % mit der Nukleotidsequenz von SEQ ID NO: 1, 2, 3, oder 4 identisch ist, aufweist, wobei das Nukleinsäuremolekül imstande ist, eine mRNA-Expression aus einem Reportervektor ohne Promotor zu regeln, oder einem Komplement davon oder einer Zelle, die das Nukleinsäuremolekül beinhaltet, und
b) Bestimmen, ob die mRNA-Expression oder das durch die cDNA codierte Polypeptid moduliert ist, und **dadurch** Identifizieren eines Antibiotikums, das ein EAAT2-Expressionsfördermittel ist.

6. Verwendung gemäß Anspruch 1, wobei das Antibiotikum eine Verbindung ist, die durch einen Screening-Assay identifiziert wird, welcher die Folgenden Schritte beinhaltet:
a) In-Kontakt-Bringen einer Zelle, die EAAT2 exprimiert, mit einem Testantibiotikum und
b) Bestimmen, ob die Expression von dem EAAT2 in der Zelle in der Gegenwart des Testantibiotikums im Vergleich zu der Abwesenheit des Testantibiotikums moduliert wird, und **dadurch** Identifizieren eines Antibiotikums, das die Expression des EAAT2 moduliert.

7. Verwendung gemäß einem der vorhergehenden Ansprüche, wobei das Antibiotikum mindestens ein strukturelles Element, das aus Heterozyklen, die mindestens ein Ringschwefelatom beinhalten, tertiären Aminen, quaternären Ammoniumsalzen, Steroiden, Polyolen, Polyketiden, Guanidin, Harnstoff oder Arsenat, ausgewählt ist, beinhaltet.

8. Verwendung gemäß einem der vorhergehenden Ansprüche, wobei das Antibiotikum die EAAT2-Produktion relativ zu einer nicht regulierten EAAT2-Produktion um 200 % oder mehr erhöht.

9. Verwendung gemäß einem der vorhergehenden Ansprüche, wobei das Antibiotikum die EAAT2-Produktion relativ zu einer nicht regulierten Produktion um 300 % oder mehr erhöht.

10. Verwendung gemäß einem der vorhergehenden Ansprüche, wobei das Antibiotikum die EAAT2-Produktion relativ zu einer nicht regulierten Produktion um 400 % oder mehr erhöht.

11. Verwendung gemäß einem der vorhergehenden Ansprüche, wobei das Antibiotikum die EAAT2-Produktion relativ zu einer nicht regulierten Produktion um 600 % oder mehr erhöht.

12. Verwendung gemäß Anspruch 1, wobei die extrazelluläre Glutamatkonzentration relativ zu einer nicht regulierten Konzentration um mindestens etwa 50 % reduziert ist.

13. Verwendung gemäß Anspruch 12, wobei die extrazelluläre Glutamatkonzentration relativ zu einer nicht regulierten Konzentration um mindestens etwa 75 % reduziert ist.

14. Verwendung gemäß einem der Ansprüche 1 bis 4, wobei das Antibiotikum ein β-Lactam-Antibiotikum ist.

15. Verwendung gemäß einem der Ansprüche 1 bis 4, wobei das Antibiotikum ein β-Lactam-Antibiotikum der Penicillinklasse, der Cephalosporinklasse, der Carbapenamklasse oder der Monobactamklasse ist.

16. Verwendung gemäß einem der Ansprüche 1 bis 3, wobei das Antibiotikum ein β-Lactam-Antibiotikum ist, das aus der Gruppe, die aus Benzylpenicillin, Procainbenzylpenicillin, Penicillin V, Penicillin-V-Kalium, Benzathin-Penicillin, Hetacillin, Cloxacillin, Carbenicillin, Flucloxacillin, Ampicillin, Ampicillinnatrium, Amoxicillin, Co-amoxiclav, Carboxypenicillin, Ticarcillin, Timentin, Tazocin, Piperacillin, Pivmecillinam, Amoxicillin-Clavulanat, Oxacillin, Bacampicillin-HCl, Nafcillinnatrium, Cefador, Cefadroxil, Cefadyl, Cefalexin, Cefamandol, Cefazolin, Cefditoren, Cefepim, Cefetamet, Cefdinir, Cefixim, Cefizox, Cefotaxim, Cefmetazol, Cefobid, Cefonicid, Cefoperazon, Cefoperazonnatrium, Cefotan, Cefotetan, Cefoxitin, Cefpirom, Cefpodoxim, Cefpodoxim-Proxetil, Cefprozil, Cefradin, Ceftazidim, Ceftibuten, Ceftidoren, Ceftin, Ceftizoxim, Ceftriaxon, Ceftriaxonnatrium, Cefuroxim, Cefuroximaxetil, Cephalexin, Cefzil, Cephalothin, Cephalothinnatrium, Cephapirinnatrium, Aztreonam, Imipenem, Meropenem, Ertapenem und FK-037 besteht, ausgewählt ist.

17. Verwendung gemäß Anspruch 16, wobei das β-Lactam-Antibiotikum aus der Gruppe, die aus Penicillin V, Penicillin-V-Kalium, Amoxicillin, Ceftriaxon, Ceftriaxonnatrium, Cepharpirinnatrium, Cefoperazon, Cefoperazonnatrium, Cefadroxil, Bacampicillin-HCl, Ampicillin, Ampicillinnatrium, Cephalothin, Cephalothinnatrium und Nafcillinnatrium besteht, ausgewählt ist.

18. Verwendung gemäß einem der Ansprüche 1 bis 4, wobei das Antibiotikum aus der Gruppe, die aus Dequaliniumchlorid, Vancomycinhydrochlorid, Actinospectacin, Oxytetracyclin, Sisomicinsulfat, Erythromycinethylsuccinat, Dihydrostreptomycinsulfat, Amprolium, Alexidin-HCl, Clindamycin, Enoxolon, Rifampin, Chloroxin, Methenamin und Pyrithionzink besteht, ausgewählt ist.

19. Verwendung gemäß Anspruch 17, wobei das Antibiotikum Ceftriaxon oder Ceftriaxonnatrium ist.

20. Verwendung gemäß einem der Ansprüche 1 bis 4, wobei das Antibiotikum Thiamphenicol ist.

21. Verwendung gemäß einem der Ansprüche 1 bis 4, wobei das Antibiotikum Chloramphenicol ist.

22. Verwendung gemäß Anspruch 19, wobei die neurologische Krankheit oder Erkrankung amyotrophe Lateralsklerose ist.

23. Ein In-Vitro-Verfahren der Erhöhung der EAAT2-Proteinexpression, das den Schritt des In-Kontakt-Bringens einer Zelle mit mindestens einem EAAT2-Expressionsfördermittel, das ein Antibiotikum ist, beinhaltet.

## Revendications

1. Utilisation d'un agent promoteur de l'expression des EAAT2 qui est un antibiotique, dans la fabrication d'un médicament destiné au traitement d'une maladie ou d'un trouble neurologique associé(e) à une transmission altérée du glutamate.

2. Utilisation selon la revendication 1, dans laquelle la maladie ou le trouble neurologique associé(e) à une transmission altérée du glutamate est associé(e) à l'apprentissage, à la mémoire ou à la cognition.

3. Utilisation selon la revendication 1 dans laquelle la maladie ou le trouble neurologique est sélectionné(e) dans le groupe constitué de la maladie de Parkinson, la maladie de Huntington, la maladie d'Alzheimer, la sclérose en plaques, la sclérose latérale amyotrophique, les maladies neurologiques aiguës, l'épilepsie, les traumatismes médullaires, les traumatismes cérébraux, les glaucomes et les troubles psychiatriques.

4. Utilisation selon la revendication 3 dans laquelle la maladie ou le trouble neurologique est la sclérose latérale amyotrophique.

5. Utilisation selon la revendication 1, dans laquelle l'antibiotique est un composé identifié par un test de criblage comprenant les étapes de :
a) mettre en contact un antibiotique d'essai avec une molécule d'acide nucléique comprenant une molécule d'ADNc et présentant une séquence nucléotidique qui est identique à au moins environ 60 % à la séquence nucléotidique de la SEQ ID NO : 1, 2, 3, ou 4, où la molécule d'acide nucléique est capable de diriger l'expression des ARNm à partir d'un vecteur rapporteur exempt de promoteur, ou un complément de celle-ci, ou une cellule comprenant ladite molécule d'acide nucléique ; et
b) déterminer si l'expression des ARNm ou le polypeptide encodé par l'ADNc est modulé(e), identifiant de ce fait un antibiotique qui est un agent promoteur de l'expression des EAAT2.

6. Utilisation selon la revendication 1, dans laquelle l'antibiotique est un composé identifié par un test de criblage comprenant les étapes de :
a) mettre en contact une cellule qui exprime des EAAT2 avec un antibiotique d'essai ; et
b) déterminer si l'expression des EAAT2 dans la cellule est modulée lorsque l'antibiotique d'essai est présent par comparaison avec lorsque l'antibiotique d'essai est absent, identifiant de ce fait un antibiotique qui module l'expression des EAAT2.

7. Utilisation selon n'importe quelle revendication précédente, dans laquelle l'antibiotique comprend au moins un élément structural sélectionné parmi des hétérocycles comprenant au moins un atome de soufre cyclique, des amines tertiaires, des sels d'ammonium quaternaire, des stéroïdes, des polyols, des polykétides, de la guanidine, de l'urée, ou de l'arséniate.

8. Utilisation selon n'importe quelle revendication précédente, dans laquelle l'antibiotique augmente de 200 % ou plus la production de EAAT2 par rapport à une production de EAAT2 non régulée.

9. Utilisation selon n'importe quelle revendication précédente, dans laquelle l'antibiotique augmente de 300 % ou plus la production de EAAT2 par rapport à une production non régulée.

10. Utilisation selon n'importe quelle revendication précédente, dans laquelle l'antibiotique augmente de 400 % ou plus la production de EAAT2 par rapport à une production non régulée.

11. Utilisation selon n'importe quelle revendication précédente, dans laquelle l'antibiotique augmente de 600 % ou plus la production de EAAT2 par rapport à une production non régulée.

12. Utilisation selon la revendication 1, dans laquelle la concentration de glutamate extracellulaire est réduite de 50 % environ au moins par rapport à une concentration non régulée.

13. Utilisation selon la revendication 12, dans laquelle la concentration de glutamate extracellulaire est réduite de 75 % environ au moins par rapport à une concentration non régulée.

14. Utilisation selon n'importe lesquelles des revendications 1 à 4, dans laquelle l'antibiotique est une β-lactamine.

15. Utilisation selon n'importe lesquelles des revendications 1 à 4, dans laquelle l'antibiotique est une β-lactamine de la classe des pénicillines, de la classe des céphalosporines, de la classe des carbapénames ou de la classe des monobactams.

16. Utilisation selon n'importe lesquelles des revendications 1 à 3, dans laquelle l'antibiotique est une β-lactamine sélectionnée dans le groupe constitué de la benzylpénicilline, de la benzylpénicilline procaïnique, de la pénicilline V, de la pénicilline V potassique, de la benzathine pénicilline, de l'hétacilline, de la cloxacilline, de la carbénicilline, de la flucloxacilline, de l'ampicilline, de l'ampicilline sodique, de l'amoxicilline, du co-amoxiclav, de la carboxypénicilline, de la ticarcilline, de la timentine, de la tazocine, de la pipéracilline, du pivmecillinam, de l'amoxicilline-clavulanate, de l'oxacilline, de la bacampicilline HCl, de la nafcilline sodique, du céfaclor, du céfadroxil, du céfadyl, de la céfalexine, du céfamandole, de la céfazoline, de la cefditorène, du céfépime, du céfétamet, du cefidinir, de la céfixime, du céfizox, du céfotaxime, du cefmétazole, du céfobide, du céfonicide, de la céfopérazone, de la céfopérazone sodique, du céfotan, du céfotétan, de la céfoxitine, du cefpirome, du cefpodoxime, du cefpodoxime proxétil, du cefprozil, de la céfradine, de la ceftazidime, du ceftibutène, de la ceftidorène, du ceftin, de la ceftizoxime, de la ceftriaxone, de la ceftriaxone sodique, du céfuroxime, du céfuroxime axétil, de la céfalexine, du cefzil, de la céphalothine, de la céphalothine sodique, de la céfapirine sodique, de l'Aztréonam, de l'imipenem, du Meropenem, de l'Ertapenem et du FK-037.

17. Utilisation selon la revendication 16, dans laquelle la β-lactamine est sélectionnée dans le groupe constitué de pénicilline V, pénicilline V potassique, amoxicilline, ceftriaxone, ceftriaxone sodique, céfapirine sodique, céfopérazone, céfopérazone sodique, céfadroxil, bacampicilline HCl, ampicilline, ampicilline sodique, céphalothine, céphalothine sodique, et nafcilline sodique.

18. Utilisation selon n'importe lesquelles des revendications 1 à 4, dans laquelle l'antibiotique est sélectionné dans le groupe constitué de cotarmine de déqualinium, chlorhydrate de vancomycine, actinospectacine, oxytétracycline, sulfate de sisomicine, éthylsuccinate d'érythromycine, sulfate de dihydrostreptomycine, amprolium, alexidine HCl, clindamycine, énoxolone, rifampine, chloroxine, méthénamine, et zinc de pyrithione.

19. Utilisation selon la revendication 17, dans laquelle l'antibiotique est de la ceftriaxone ou de la ceftriaxone sodique.

20. Utilisation selon n'importe lesquelles des revendications 1 à 4, dans laquelle l'antibiotique est du thiamphénicol.

21. Utilisation selon n'importe lesquelles des revendications 1 à 4, dans laquelle l'antibiotique est du chloramphénicol.

22. Utilisation selon la revendication 19 dans laquelle la maladie ou le trouble neurologique est la sclérose latérale amyotrophique.

23. Une méthode in vitro pour augmenter l'expression des protéines EAAT2 comprenant l'étape de mettre en contact une cellule avec au moins un agent promoteur de l'expression des EAAT2 qui est un antibiotique.
